# EUROPEAN PATENT APPLICATION

(11) **EP 4 397 307 A1**
(43) Date of publication of application: **10.07.2024**
(21) Application number: 22865008.1
(22) Date of filing: 30.08.2022
(51) Int. Cl.: A61K 31/4965, A61K 31/497, A61K 45/06, A61P 35/00, G01N 33/50, G01N 33/68, G01N 33/58

(54) **CANCER IMMUNOTHERAPY COMPOSITION CONTAINING INTERACTION INHIBITOR OF STING AND TRIM29 AS ACTIVE INGREDIENT**

(30) Priority: 31.08.2021 KR 20210115253
(71) Applicant: Spark Biopharma, Inc., Seoul 08791 (KR)
(72) Inventor: PARK, Seung Bum, Seoul 04427 (KR); CHO, Wan Sang, Daejeon 35218 (KR)
(74) Representative: Schön, Christoph
(86) International application number: PCT/KR2022/012938
(87) International publication number: WO 2023/033506

(57) **Abstract**

The present invention relates to a cancer immunotherapy composition containing, as an active ingredient, an interaction inhibitor of stimulator of interferon genes (STING) and tripartite motif-containing protein 29 (TRIM29). Specifically, the interaction inhibitor of STING and TRIM29 according to the present invention inhibits STING degradation due to TRIM29 and increases the intracellular amount of STING, thereby further activating immune response mediated by the STING agonist cGAMP. Furthermore, the interaction inhibitor of STING and TRIM29 exhibits significant immune anticancer efficacy when administered in combination with the STING agonist cGAMP and an immune checkpoint inhibitor and can thus be effectively used as a cancer immunotherapy agent and a cancer immunotherapy adjuvant.

## Description

### [Technical Field]

The present invention relates to a cancer immunotherapy agent containing an interaction inhibitor of stimulator of interferon genes (STING) and tripartite motif-containing protein 29 (TRIM29) as an active ingredient and a cancer immunotherapy adjuvant.

### [Background Art]

Cancer is generally a disease in which cells do not differentiate normally and continue to divide due to abnormalities in a cycle of cells that form human tissues and occurs through three stages of initiation, promotion, and progression. It is known that the cause of cancer is that mutations occur in genes of normal cells or tumor inhibitory genes by carcinogens included in the environment or food, and these cells are continuously stimulated by the carcinogens thereby abnormally proliferating and forming cancerous tissue.

Most of treatments for cancer include surgical operations, radiation therapies, and immuno-anticancer therapy along with chemotherapy, and methods for researching anticancer drugs to treat cancer include a method for searching for cytotoxic substances directly against cancer cells, a method for searching for substances that regulate immune capacity of living organisms, a method for searching for substances that inhibit metastasis of cancer cells, and a method for searching for substances that inhibit angiogenesis. However, most of the anticancer drugs currently used in clinical practice are chemically synthesized substances that cause problems such as causing toxicity to normal cells, and treatments using immune responses are recently attracting attention to solve these problems.

Meanwhile, stimulator of interferon genes (STING) plays a central role in DNA-sensing antiviral immunity. Specifically, when abnormal cytoplasmic DNA is detected, cGAMP (cyclic GMP-AMP), which acts as an innate immune agent, is produced by cGAMP synthase (cGAS), and cGAMP activates STING, which is an immune receptor. Additionally, activated STING induces the production of type I interferons and pro-inflammatory cytokines to recruit adaptive immune cells to eliminate damaged host cells. Therefore, STING acts as a central interface between innate immunity and acquired immunity. In practice, recent studies have shown that intratumoral administration of STING agonists activates local immunity in the tumor microenvironment (TME) and recruits effector cells to eliminate cancer.

Also, tripartite motif-containing protein 29 (TRIM29) E3 ligase is a direct regulator of STING, and TRIM29-mediated STING ubiquitination degrades STING in both host immune cells and cancer cells in a K48 linkage-specific manner. Under normal conditions, TRIM29 promotes non-immunogenic degradation of STING, and in contrast, overexpression of TRIM29 interferes with immune responses and reduces cytokine production when treating with STING agonists. However, many studies report that direct inhibition of TRIM29 causes cancer metastasis. TRIM29 is a highly downregulated gene in metastatic melanoma, and loss of TRIM29 increases the metastatic profile of squamous cell carcinoma by disrupting keratin distribution. However, it has not yet been shown that specific perturbation of the protein-protein interaction (PPI) between STING and TRIM29 can overcome the weakened efficacy of STING agonists by increasing cellular STING levels without potential metastatic risk.

Accordingly, the present inventors have diligently conducted research to develop a cancer immunotherapy agent that inhibits the STING-TRIM29 interaction and prevents the degradation of STING due to TRIM29, have resultantly constructed a high-efficiency biological activity search method that can screen compounds that inhibit the interaction of STING and TRIM29, and have discovered oxopiperazine derivatives through the constructed system. In addition, the present inventors have confirmed that the oxopiperazine derivative can further activate immune response mediated by the STING agonist cGAMP by increasing the intracellular amount of STING, thereby increasing the immune anticancer efficacy of cGAMP and immune checkpoint inhibitor anti-PD-1 therapy. Accordingly, the present inventors have discovered that an interaction inhibitor of STING and TRIM29 according to the present invention can be usefully used as an active ingredient in cancer immunotherapy agents and cancer immunotherapy adjuvants to complete the present invention.

### [Citation List]

### [Non Patent Literature]

[Non Patent Literature 1]
   Q. Chen, L. Sun, Z. J. Chen, Regulation and function of the cGAS-STING pathway of cytosolic DNA sensing. Nat. Immunol. 17, 1142-1149 (2016).
[Non Patent Literature 2]
   L. Sun, J. Wu, F. Du, X. Chen, Z. J. Chen, Cyclic GMP-AMP Synthase Is a Cytosolic DNA Sensor That Activates the Type I Interferon Pathway. Science. 339, 786-791 (2013).

### [Summary of Invention]

### [Technical Problem]

An object of the present invention is to provide an interaction inhibitor of stimulator of interferon genes (STING) and tripartite motif-containing protein 29 (TRIM29), and the use for cancer immunotherapy.

Another object of the present invention is to provide a method and kit for screening for discovering an interaction inhibitor of stimulator of interferon genes (STING) and tripartite motif-containing protein 29 (TRIM29) .

### [Solution to Problem]

In order to achieve the above object, the present invention provides a cancer immunotherapy composition containing an interaction inhibitor of stimulator of interferon genes (STING) and tripartite motif-containing protein 29 (TRIM29) as an active ingredient.

The present invention provides a cancer immunotherapy composition containing oxopiperazine derivatives, isomers thereof, solvates thereof, hydrates thereof, or pharmaceutically acceptable salts thereof as an active ingredient.

Also, the present invention provides a cancer immunotherapy adjuvant containing the interaction inhibitor of STING and TRIM29, isomers thereof, solvates thereof, hydrates thereof, or pharmaceutically acceptable salts thereof as an active ingredient.

Also, the present invention provides an anticancer combination agent containing the cancer immunotherapy agent and the cancer immunotherapy adjuvant.

Also, the present invention provides a method for screening cancer immunotherapy agent, including:
1) a step of preparing a vector including genes encoding a STING protein bound to a luminescent protein and a TRIM29 protein bound to a luminescent protein, respectively;
2) a step of transforming the vector of step 1) into a cell;
3) a step of treating the transformed cell of step 2) with a test substance;
4) a step of comparing the cell treated with the test substance of step 3) with an untreated control group and selecting a substance that reduces the luminescence signal from the treated cell.

In addition, the present invention provides a kit for screening cancer immunotherapy agent, including a STING protein bound to a luminescent protein and a TRIM29 protein bound to a luminescent protein.

### [Advantageous Effects of Invention]

An interaction inhibitor of stimulator of interferon genes (STING) and tripartite motif-containing protein 29 (TRIM29) according to the present invention further activates immune response mediated by the STING agonist cGAMP by inhibiting the degradation of STING due to TRIM29 and increasing the intracellular amount of STING, and further exhibits significant immune anticancer efficacy when administered in combination with the STING agonist cGAMP and immune checkpoint inhibitor. Therefore, the interaction inhibitor of STING and TRIM29 can be usefully used as a cancer immunotherapy agent and a cancer immunotherapy adjuvant.

### [Brief Description of Drawings]

FIG. 1 is a schematic diagram of a screening system of the present invention for screening a compound that inhibit interaction of stimulator of interferon genes (STING) and tripartite motif-containing protein 29 (TRIM29) .
FIG. 2 is a diagram illustrating confirmation of an optimized screening system by using a total of eight combinations obtained by introducing NanoBiT (LgBiT and SmBiT) to each of N-terminuses and C-terminuses of STING and TRIM29:
   NL: a group obtained by introducing LgBiT to an N-terminus;
   NS: a group obtained by introducing SmBiT to an N-terminus;
   CL: a group obtained by introducing LgBiT to a C-terminus; and
   CS: a group obtained by introducing SmBiT to a C-terminus.
FIG. 3 is a diagram illustrating an effect of an oxopiperazine derivative in reducing a luminescence signal through Luciferase Complementary Assay by inhibiting the STING-TRIM29 interaction:
   1: Formula 1 of the present invention (hereinafter, a compound of Formula 1 is indicated as "1" in the drawings of the present invention,);
   2: Formula 2 of the present invention (hereinafter, a compound of Formula 2 is indicated as "2" in the drawings of the present invention,);
   3: Formula 3 of the present invention (hereinafter, a compound of Formula 3 is indicated as "3" in the drawings of the present invention,);
   4: Formula 4 of the present invention (hereinafter, a compound of Formula 4 is indicated as "4" in the drawings of the present invention,);
   5: Formula 5 of the present invention (hereinafter, a compound of Formula 5 is indicated as "5" in the drawings of the present invention,); and
   6: Formula 6 of the present invention (hereinafter, a compound of Formula 6 is indicated as "6" in the drawings of the present invention,).

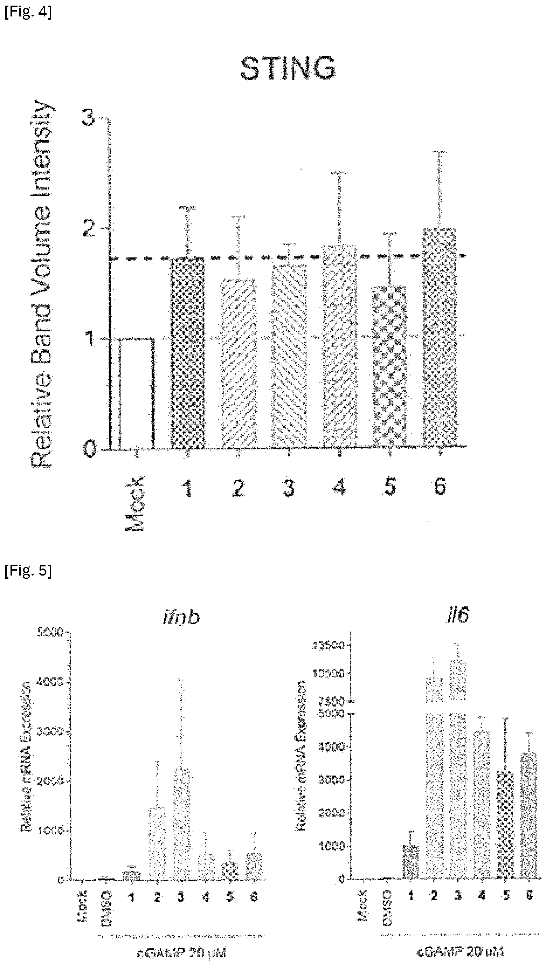

FIG. 4 is a diagram illustrating confirmation of an effect of an oxopiperazine derivative on inducing an increase in intracellular amount of STING protein.
FIG. 5 is a diagram illustrating an effect of increasing expression of IFN-β and IL-6, which are STING subcytokine, under co-treatment with the oxopiperazine derivative of the present invention and cGAMP.

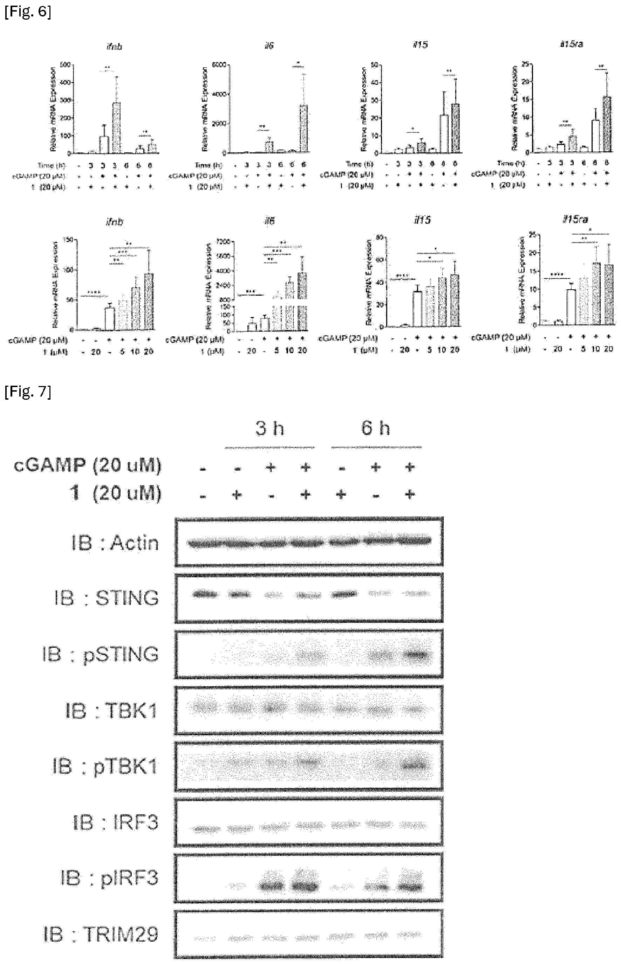

FIG. 6 is a diagram illustrating time-dependent/dose-dependent confirmation of an effect of the compound of Formula 1 on increasing expression of IL-15 and IL-15Rα, which are STING subcytokine.
FIG. 7 is a diagram illustrating confirmation of an effect of the compound of Formula 1 on increasing activation of a STING sub-immune signaling pathway through Western blotting.

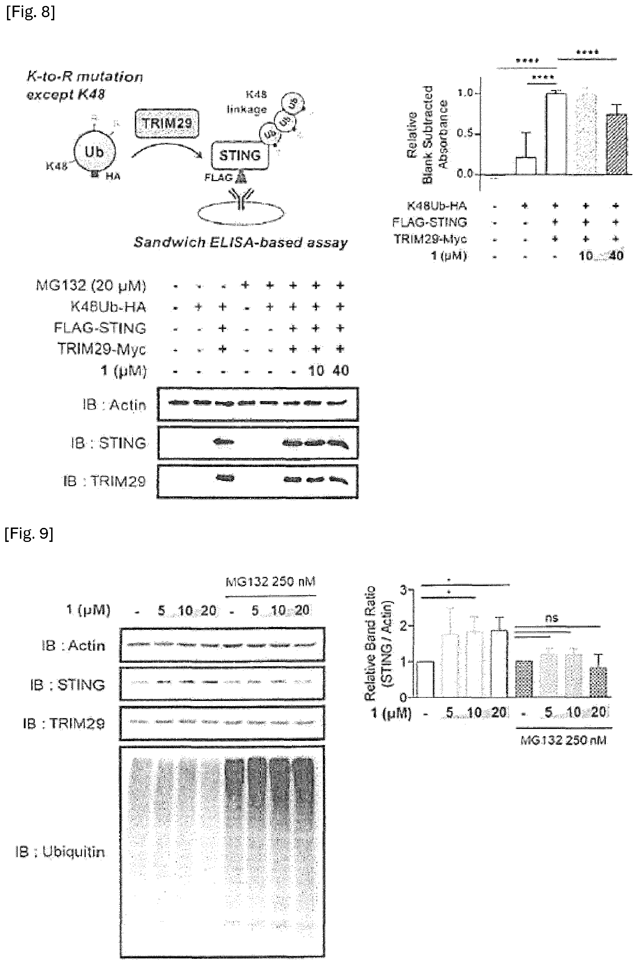

FIG. 8 is a diagram illustrating confirmation of dose-dependent reduction of Lys48 polymerized multi-ubiquitination of STING due to the compound of Formula 1 as a result of quantitative STING Lys48 polymerized multi-ubiquitination detection based on the ELISA (Enzyme-linked immunosorbent assay) method by using a transgenic ubiquitin protein obtained by replacing all lysine residues other than lysine residue 48 in ubiquitin protein with arginine.
FIG. 9 is a diagram illustrating confirmation on whether the STING increasing effect of the compound of Formula 1 disappears when ubiquitinated proteins are prevented from being degraded by treatment with MG132, which is a proteasome inhibitor.

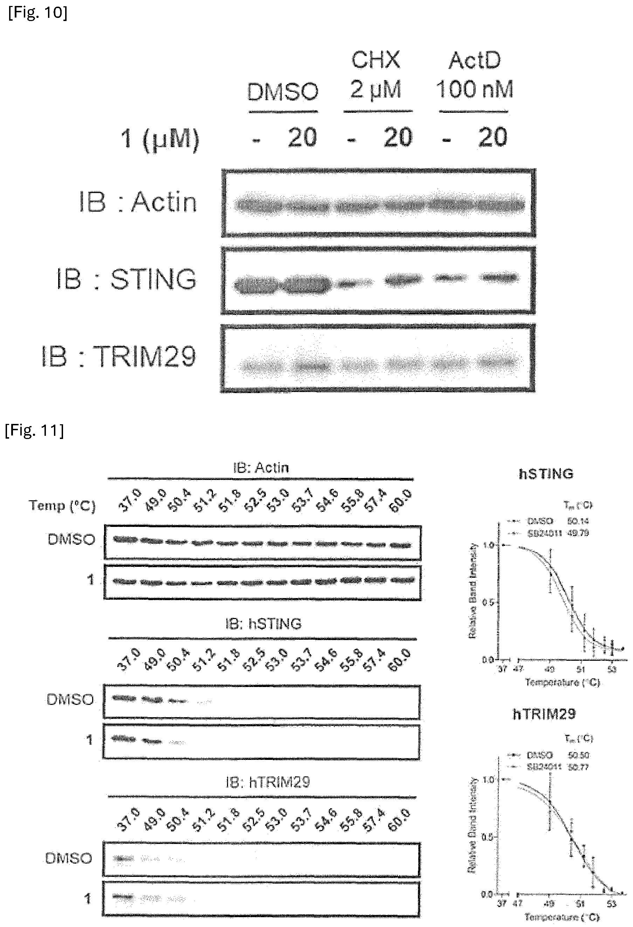

FIG. 10 is a diagram illustrating confirmation on whether the STING expression increasing effect of the compound of Formula 1 is exhibited through a different cellular mechanism other than the ubiquitin-mediated reaction to confirm that the STING expression increasing effect of the compound is not inhibited by a protein transcription inhibitor (cyclohexamide, CHX) and a transcription inhibitor (actinomycin D, ActD).
FIG. 11 is a diagram illustrating confirmation that the compound of Formula 1 induces thermal destabilization by selectively binding only to STING through CETSA results in A431, which is a human cell line.

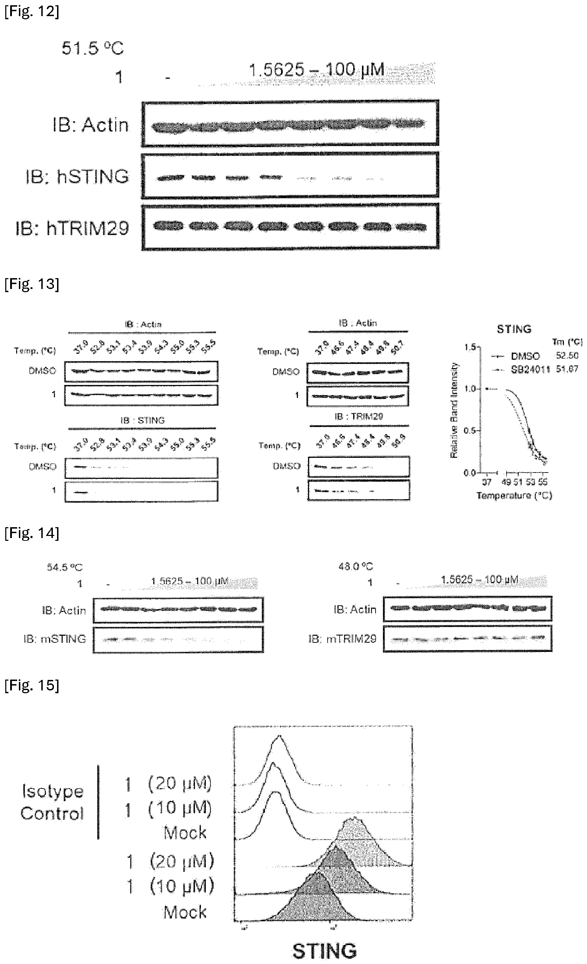

FIG. 12 is a diagram illustrating cross-verification of concentration-dependent thermal destabilization of STING by the compound of Formula 1 at high concentrations without affecting TRIM29 through ITDRF results in A431, which is a human cell line.
FIG. 13 is a diagram illustrating confirmation that the compound of Formula 1 induces thermal destabilization by selectively binding only to STING through CETSA results in Raw 264.7, which is a mouse cell line.
FIG. 14 is a diagram illustrating cross-verification of concentration-dependent thermal destabilization of STING by the compound of Formula 1 without affecting TRIM29 at high concentrations through ITDRF results in Raw 264.7, which is a mouse cell line.
FIG. 15 is a diagram illustrating confirmation of an fluorescence labeling intensity of intracellular STING by using flow cytometry when Raw 264.7 cells are treated with the compound of Formula 1.

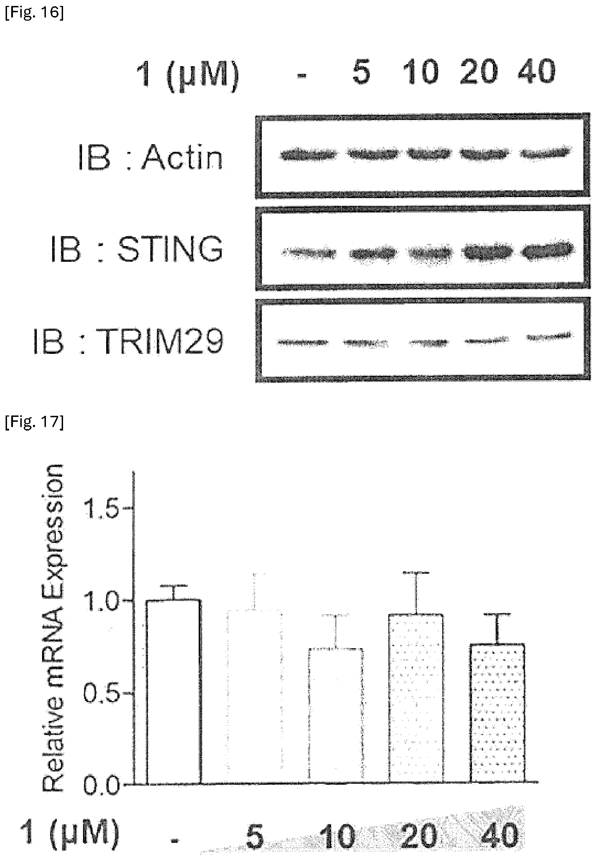

FIG. 16 is a diagram illustrating confirmation of an intracellular amount of STING through Western blotting analysis when Raw 264.7 cells are treated with the compound of Formula 1.
FIG. 17 is a diagram illustrating confirmation of an intracellular mRNA expression amount of STING through the qRT-PCR method when Raw 264.7 cells are treated with the compound of Formula 1.

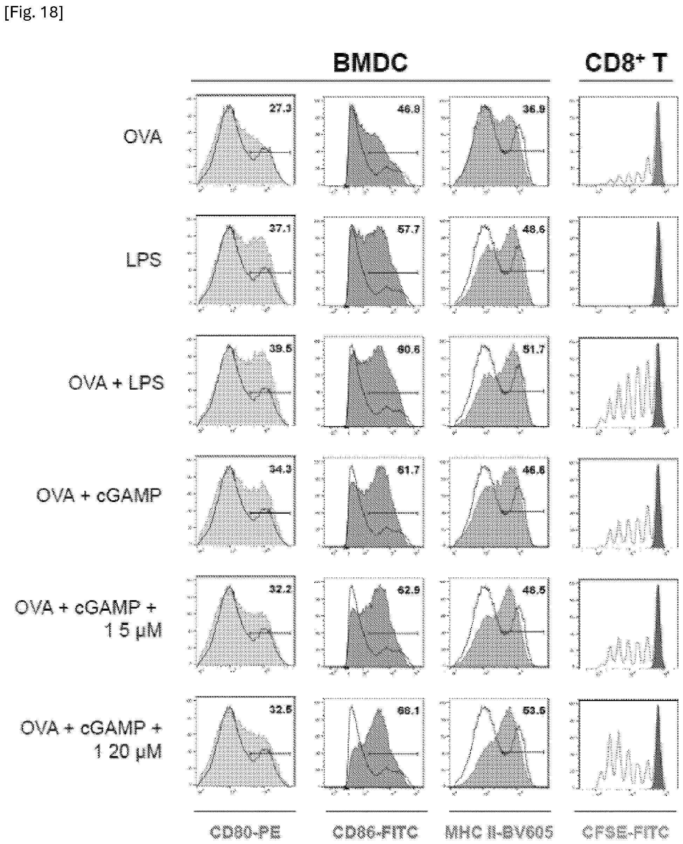

FIG. 18 is a diagram illustrating confirmation of an effect of increasing antigen presentation efficacy of BMDC due to STING agonist cGAMP of the compound of Formula 1. Also, FIG. 18 is a diagram illustrating confirmation that the proliferation of cytotoxic T cells increases by the compound. It is confirmed that the these effects of the compound showed synergy when administered in combination with cGAMP.

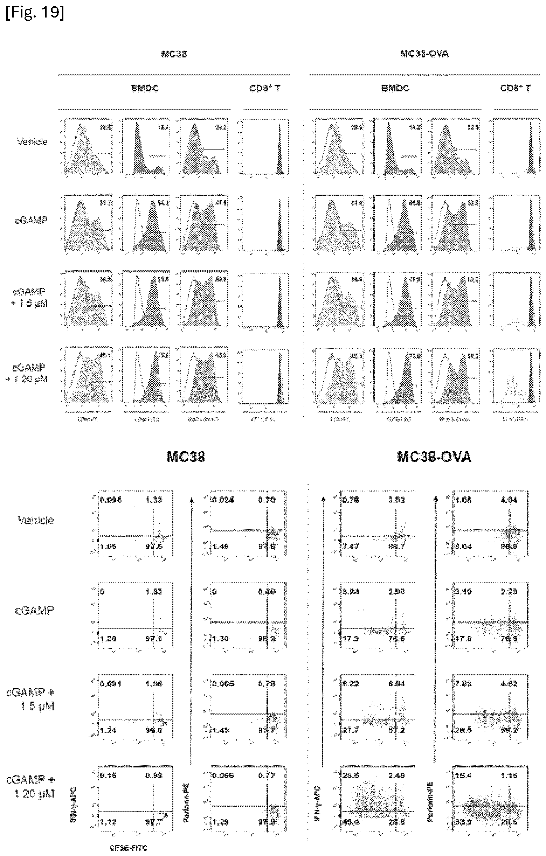

FIG. 19 is a diagram illustrating confirmation of cross-antigen presentation ability of BMDC by simultaneous treatment of the compound of Formula 1 and cGAMP.

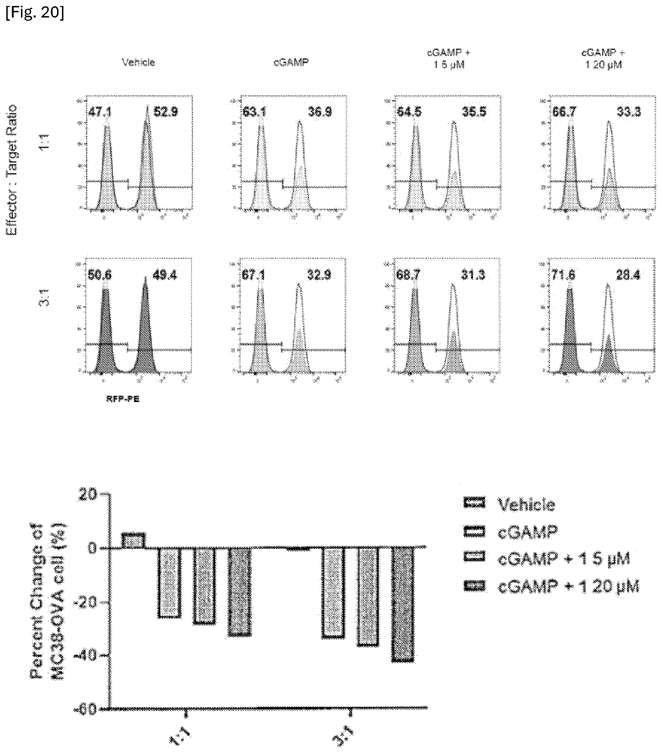

FIG. 20 is a diagram illustrating confirmation that activated OT-1 T cells selectively kill only cell lines expressing OVA through the experiment of FIG. 19.

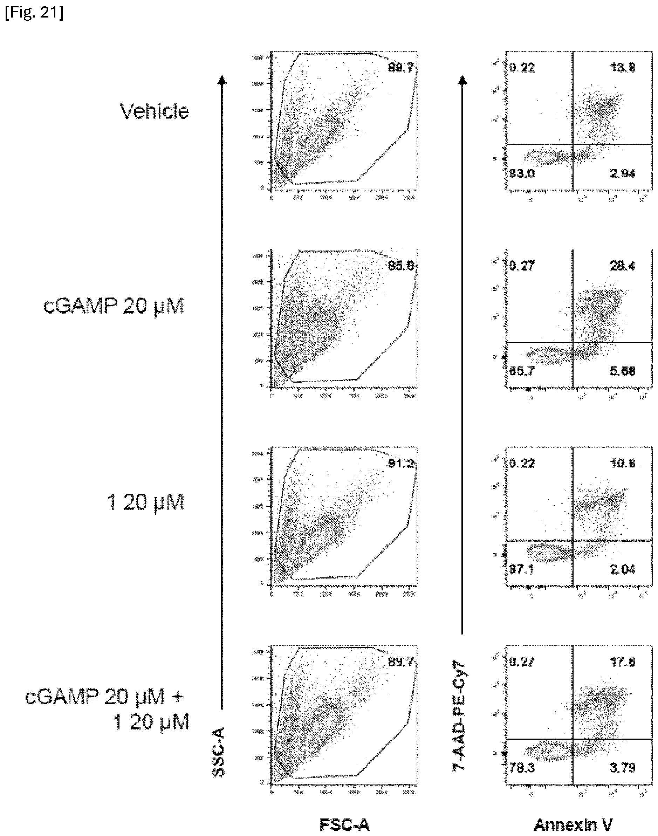

FIG. 21 is a diagram illustrating confirmation that death of cytotoxic T cells is not induced or promoted under the conditions of treating the compound of Formula 1 alone or in combination with cGAMP.

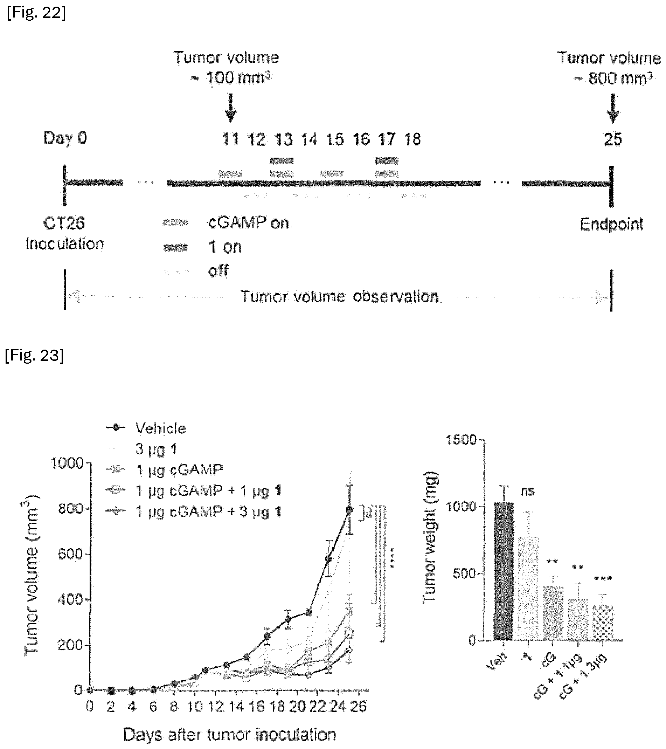

FIG. 22 is a schematic diagram illustrating an animal testing method to confirm the immune anticancer efficacy of cGAMP by the compound of Formula 1 in an *in vivo* syngeneic mouse model.
FIG. 23 is a diagram illustrating confirmation of a tumor growth inhibitory effect in an experimental group of a combination treatment of cGAMP and the compound of Formula 1 in the *in vivo* syngeneic mouse model.

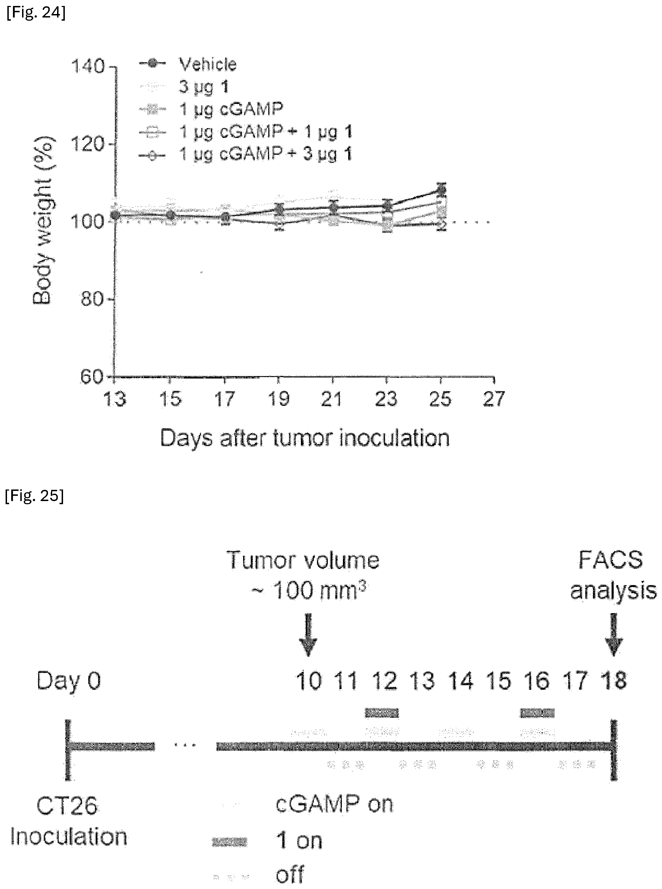

FIG. 24 is a diagram illustrating confirmation of body weight changes and toxicity signals in the experimental group of the combination treatment of cGAMP and the compound of Formula 1 in the *in vivo* syngeneic mouse model.
FIG. 25 is a diagram illustrating an experimental procedure for verifying immune cell infiltration into a tumor tissue and a degree of activation of an infiltrated immune cell.

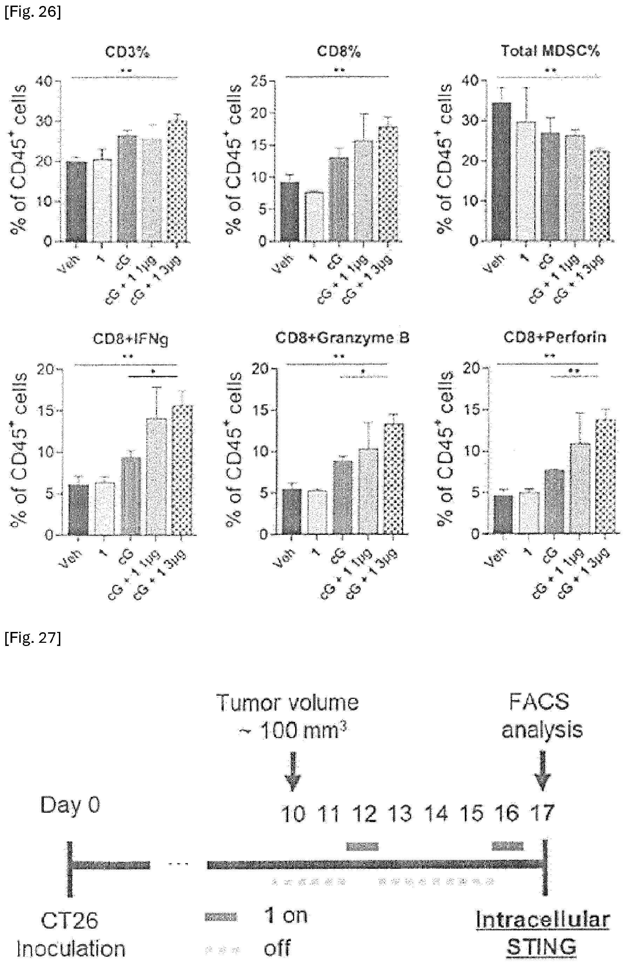

FIG. 26 is a diagram illustrating confirmation of the immune cell infiltration into a tumor tissue and a degree of activation of the infiltrated immune cell after treatment with cGAMP and the compound of Formula 1 alone or in combination.
FIG. 27 is a diagram illustrating an experimental procedure for confirming an expression level of STING in an immune cell inside a tumor.

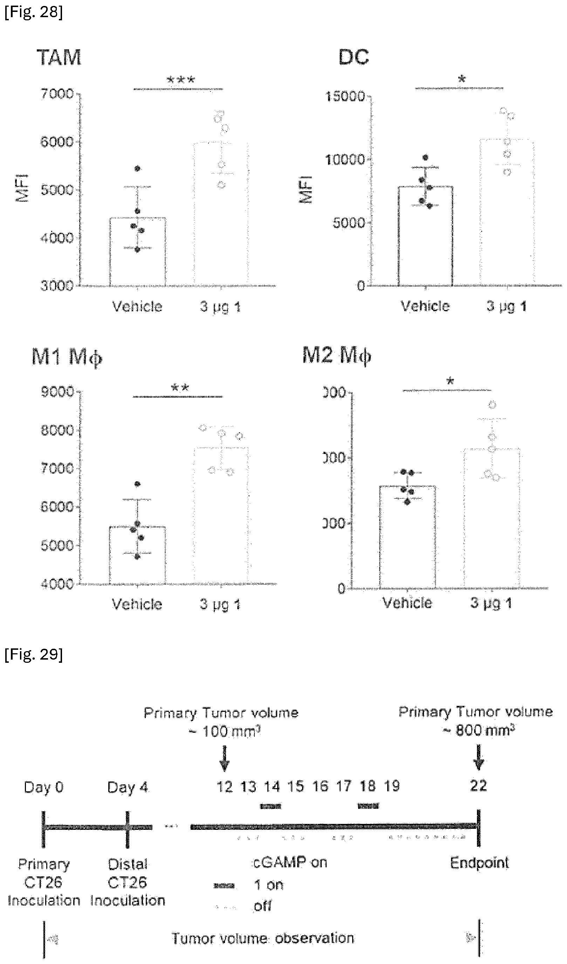

FIG. 28 is a diagram illustrating confirmation of an expression level of STING in an immune cell inside a tumor according to treatment of the compound of Formula 1.
FIG. 29 is a diagram illustrating an experimental procedure for confirming an anticancer immune system construction effect of the compound of Formula 1.

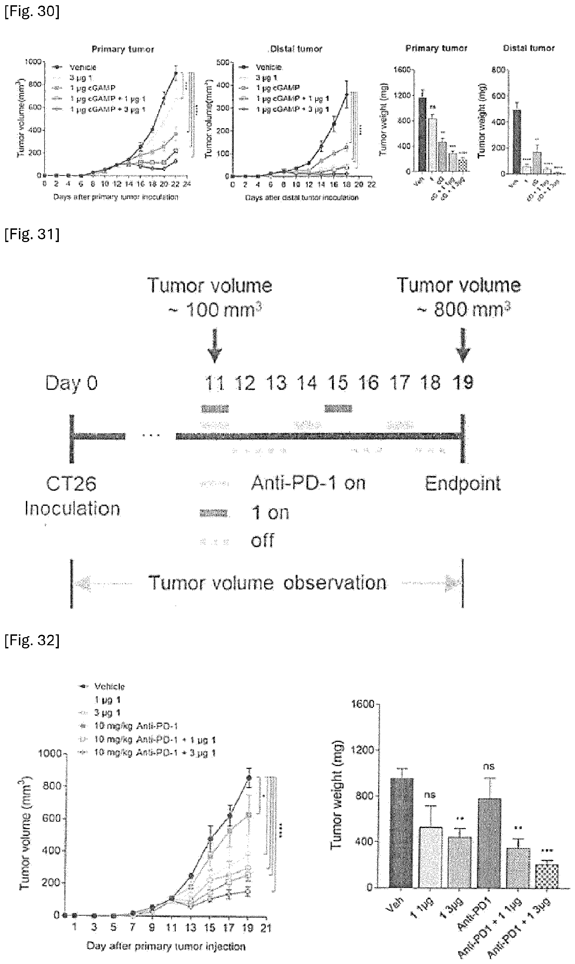

FIG. 30 is a diagram illustrating confirmation of an anticancer immune system construction promotion effect of the compound of Formula 1.
FIG. 31 is a diagram illustrating an experimental procedure for confirming an effect of the combination treatment of the compound of Formula 1 and the immune checkpoint therapeutic agent.
FIG. 32 is a diagram illustrating confirmation of an effect of the combination treatment of the compound of Formula 1 and the immune checkpoint therapeutic agent.

### [Description of Embodiments]

Hereinafter, the present invention is described in detail.

The present invention provides a cancer immunotherapy composition containing an interaction inhibitor of stimulator of interferon genes (STING) and tripartite motif-containing protein 29 (TRIM29) as an active ingredient.

The interaction inhibitor is preferably a low-molecular-weight compound, peptide, or antibody, but is not limited thereto, and may be a compound represented by Formula 7, isomers thereof, solvates thereof, hydrates thereof, or pharmaceutically acceptable salts thereof: 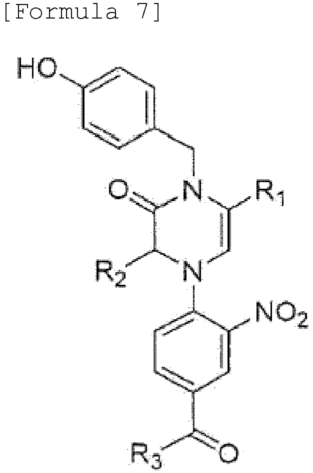
(In Formula 7, R₁ is cycloalkyl, specifically, C₃-C₇ cycloalkyl, aryl, specifically, C₆-C₁₀ aryl, or aryl-alkyl, specifically, C₆-C₁₀ aryl-C₁-C₅ alkyl, where alkyl may be optionally substituted with alkoxy;
R₂ is alkyl, or aryl-alkyl, specifically C₆-C₁₀ aryl-C₁-C₅ alkyl, where aryl may be optionally substituted with halogen; and
R₃ is amino, alkylamino, di-alkylamino, or heterocycloalkyl-alkylene-amino.

In addition, [Formula 7] is preferably a compound represented by [Formula 1] to [Formula 6] but is not limited thereto: 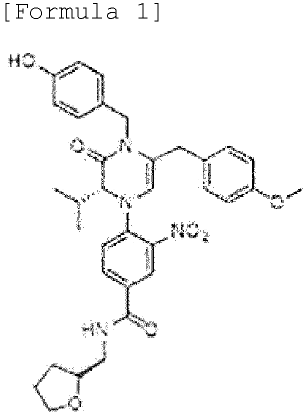 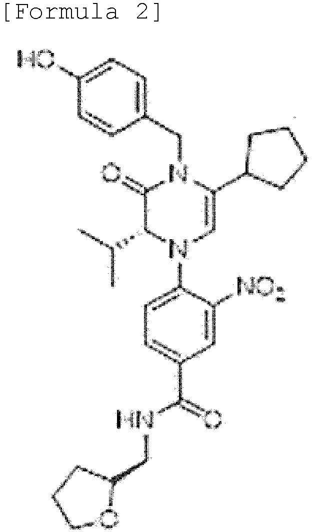 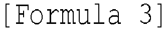 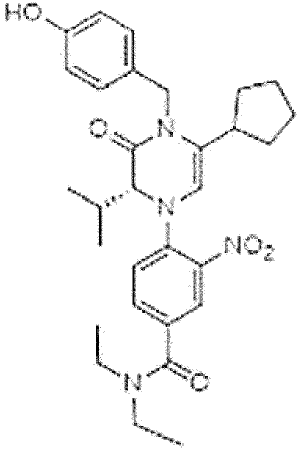 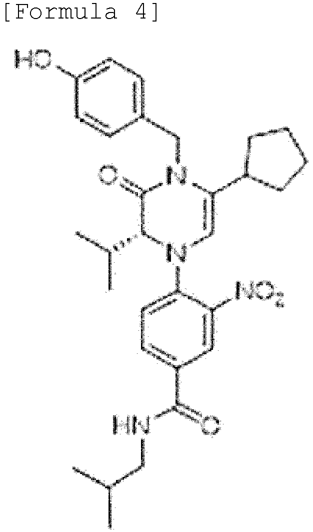 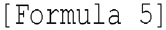 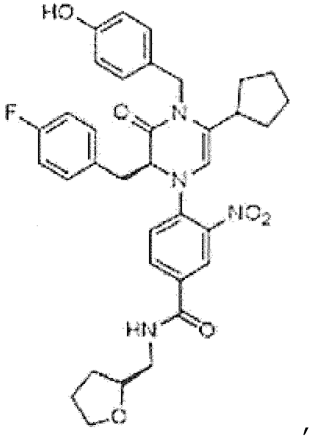 and

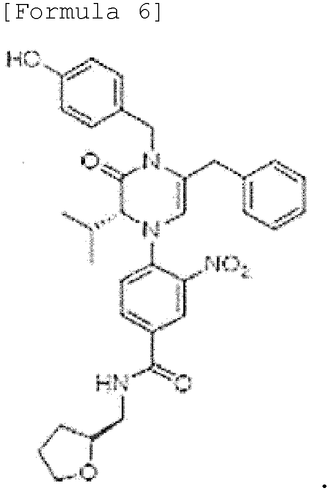

It is preferable that the interaction inhibitor binds to STING and inhibits STING degradation through TRIM29-mediated Lys48 polymerized multi-ubiquitination of STING (K48 linkage-specific polyubiquitination), but is not limited thereto.

In addition, the interaction inhibitor preferably upregulates STING and increases the activity of a STING agonist, and the STING agonist is preferably c-di-GMP (cyclic diguanylate), cGAMP, and 3'3'-cGAMP, c-di-GAMP, c-di-AMP, or 2'3'-cGAMP, but is not limited thereto.

In addition, the interaction inhibitor preferably activates one or more immune factors selected from the group consisting of cytotoxic T cells, helper T cells, NK cells, and cytokines, to have a preventive or therapeutic effect on one or more cancers selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, triple negative breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic Schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, blood cancer, and thymic cancer, but the present invention is not limited thereto.

Accordingly, it is preferable that the oxopiperazine derivative represented by [Formula 1] to [Formula 6] inhibits interaction of STING and TRIM29, increases an amount of STING protein, and inhibits STING degradation due to TRIM29-mediated Lys48 polymerized multi-ubiquitination of STING, but the present invention is not limited thereto.

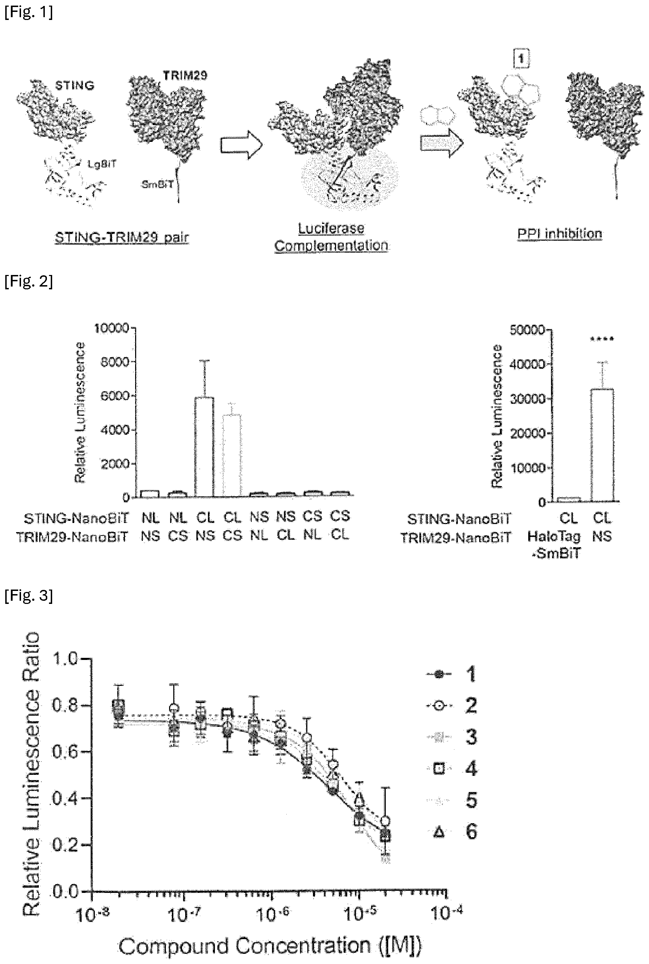

In a specific embodiment of the present invention, in order to screen a compound that inhibits the interaction of STING and TRIM29, the present inventors have developed a novel upregulation of target proteins by protein-protein interaction strategy (UPPRIS) of binding LgBiT to the C-terminus of STING and SmBiT luminescent proteins to the N-terminus or the C-terminus of TRIM29 (see FIGS. 1 and 2).

In addition, the present inventors have confirmed oxopiperazine derivatives represented by [Formula 1] to [Formula 6] that inhibit STING-TRIM29 interaction by using the above-established screening system (see FIG. 3) .

In addition, the present inventors have investigated the effect of oxopiperazine derivatives on inducing increase of an intracellular amount of STING protein, thereby confirming that all oxopiperazine derivatives significantly increase the intracellular amount of STING (see FIG. 4).

In addition, the present inventors have investigated the effect of oxopiperazine derivatives on increasing the immune activation efficacy of the STING agonist (cGAMP), thereby confirming that all oxopiperazine derivatives increase STING subcytokine expression and increase phosphorylation of downstream signaling protein under co-treatment with cGAMP (see FIGS. 6 and 7).

Also, the present inventors have investigated the mechanism of STING degradation inhibition by oxopiperazine derivatives, thereby confirming that oxopiperazine derivatives prevent STING degradation due to TRIM29-mediated Lys48 polymerized multi-ubiquitination of STING (K48 linkage-specific polyubiquitination) to increase the intracellular amount (see FIGS. 8 to 10).

Therefore, the present inventors have investigated whether the oxopiperazine derivatives bind to STING, thereby confirming that the oxopiperazine derivatives bind to STING among STING and TRIM29 and inhibits the interaction of STING and TRIM29 (see FIGS. 11 to 13).

Also, in order to confirm the effect of the oxopiperazine derivatives on increasing the efficacy of STING agonists and other cancer immunotherapy, the present inventors have first investigated whether the oxopiperazine derivatives have increased the amount of STING, thereby confirming that the intracellular amount of STING have significantly increased in a group treated with the oxopiperazine derivatives (see FIG. 15).

In addition, the present inventors have investigated the effect of the oxopiperazine derivatives on increase of antigen presentation efficacy of bone-marrow derived dendritic cells (BMDC) by STING agonists, thereby confirming that the oxopiperazine derivatives increase the antigen presentation efficacy of BMDC of the STING agonist to cytotoxic T cells (CD8⁺ T cell) (see FIG. 18).

Also, the present inventors have investigated a cross-antigen presentation ability of BMDC according to simultaneous treatment of the oxopiperazine derivatives and cGAMP, thereby confirming that the compound of Formula 1 promotes cell proliferation by increasing, in a concentration-dependent manner, the activation of BMDC by cGAMP and the antigen presentation efficacy from BMDC to OT-1 T cells when killing and treating the MC38 cell line (MC38-OVA) that continuously expresses OVA antigen and the control MC38 cell lines (see FIG. 19) and that OT-1 T cells selectively kill only cell lines expressing OVA (see FIG. 20).

Also, the present inventors have performed T cell death analysis when treating CD8⁺ T cells with cGAMP and/or oxopiperazine derivatives, thereby confirming that death of cytotoxic T cells are not induced nor promoted when treating with oxopiperazine derivatives alone or in combination with cGAMP compared with the treatment with cGAMP (see FIG. 21).

In addition, the present inventors first have investigated the tumor growth inhibitory effect in order to confirm the effect of increasing the immune anticancer efficacy of cGAMP by the oxopiperazine derivatives in an *in vivo* syngeneic mouse model, thereby confirming that tumor growth is inhibited in an experimental group of administering in combination with cGAMP and the oxopiperazine derivatives, and toxicity signals are not observed (see FIGS. 23 and 24).

In addition, the present inventors have investigated the effect of the oxopiperazine derivatives in increasing immunity activation and immune anticancer efficacy, thereby confirming that the overall immune response environment is activated by confirming that the infiltration of total T cells and cytotoxic T cells into a tumor is increased under the condition of administering in combination with cGAMP and the oxopiperazine derivatives and the total ratio of myeloid-derived suppressor cells (MDSC), which are cells that inhibit the immune response, is reduced (see FIGS. 25 and 26).

In addition, the present inventors have verified that STING increase in immune cells inside a tumor by the oxopiperazine derivatives is at the basis of increase in the immune activation and the immune anticancer efficacy, thereby confirming that the amount of STING is increased in antigen presenting cells represented by tumor-associated macrophages (TAM) and dendritic cells (DC) (see FIGS. 27 and 28).

Also, the present inventors have investigated the anticancer immune system construction effect, thereby confirming that tumor growth is inhibited in primary tumors in a concentration-dependent manner when administered in combination with the oxopiperazine derivatives, compared with single administration of cGAMP, and the final tumor mass is reduced. Furthermore, the present inventors have finally confirmed that tumor death also occurred effectively in secondary carcinomas into which the drug is not directly injected (see FIGS. 29 and 30).

In addition, the present inventors have investigated the effect of administering in combination with the immune checkpoint therapeutic agent, thereby confirming that, in case of administering in combination PD-1 antibody and the oxopiperazine derivatives, the tumor growth and the final tumor weight are increased in a concentration-dependent manner, compared with a case of administering PD-1 antibody alone (see FIGS. 31 and 32).

Therefore, since the interaction inhibitor of STING and TRIM29 according to the present invention inhibits the degradation of STING due to TRIM29 by inhibiting the STING-TRIM29 interaction, further activates immune response mediated by the STING agonist cGAMP by increasing the intracellular amount of STING, and also exhibits significant immune anticancer efficacy when administered in combination with the STING agonist cGAMP and the immune checkpoint inhibitor, the interaction inhibitor of STING and TRIM29 can be usefully used as a cancer immunotherapy agent and a cancer immunotherapy adjuvant.

The compound represented by Formula 7 of the present invention can be used in the form of a pharmaceutically acceptable salt, and an acid addition salt formed by a pharmaceutically acceptable free acid is useful as the salt. The acid addition salt is obtained from inorganic acids such as hydrochloric acid, nitric acid, phosphoric acid, sulfuric acid, hydrobromic acid, hydroiodic acid, nitrous acid, and phosphorous acid, non-toxic organic acid such as aliphatic mono- and di-carboxylates, phenyl-substituted alkanoate, hydroxy alkanoate, alkane dioate, aromatic acids, and aliphatic and aromatic sulfonic acids, and organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methane sulfonate, propane sulfonate, naphthalene-1-sulfonate, naphthalene-2-sulfonate, and mandelate.

The acid addition salt according to the present invention can be prepared by a method in the related art, and for example, may be prepared by dissolving the oxopiperazine derivative of Formula 7 in an organic solvent such as methanol, ethanol, acetone, methylene chloride, and acetonitrile, adding an organic acid or inorganic acid thereto, and filtering and drying a resulting precipitate or may be prepared by distilling a solvent and excess acid under reduced pressure, drying the resultant, and crystallizing the dried resultant in an organic solvent.

Additionally, a pharmaceutically acceptable metal salt can be prepared using a base. The alkali metal or alkaline earth metal salt is obtained, for example, by dissolving a compound in an excess of alkali metal hydroxide or alkaline earth metal hydroxide solution, filtering insoluble compound salt, and evaporating and drying the filtrate. At this time, it is pharmaceutically appropriate to prepare sodium, potassium, or calcium salts as metal salts. Additionally, the corresponding salt is obtained by reacting alkali metal or alkaline earth metal salt with a suitable negative salt (for example, silver nitrate).

Furthermore, the present invention includes not only the compound represented by Formula 7 and pharmaceutically acceptable salts thereof but also solvates, optical isomers, hydrates, and the like that can be prepared therefrom.

The term "hydrate" in the present invention refers to the compound of the present invention or salts thereof including a stoichiometric or non-stoichiometric amount of water bound by non-covalent intermolecular forces. The hydrate of the compound represented by Formula 7 of the present invention may include a stoichiometric or non-stoichiometric amount of water bound by non-covalent intermolecular forces. The hydrate may contain more than 1 equivalent of water, preferably 1 to 5 equivalents of water. Such hydrates can be prepared by crystallizing the compound represented by Formula 7 of the present invention, isomers thereof, or pharmaceutically acceptable salts thereof from water or a solvent containing water.

Additionally, the term "solvate" in the present invention refers to the compound of the present invention or a salt thereof that includes a stoichiometric or non-stoichiometric amount of solvent bound by non-covalent intermolecular forces. Preferred solvents therefor include solvents that are volatile, non-toxic, and/or suitable for administration to humans.

In addition, the term "isomer" according to the present invention refers to a compound of the present invention or a salt thereof that has the same chemical formula or molecular formula but is structurally or sterically different. These isomers include all of structural isomers such as tautomers, stereoisomers such as R or S isomers with asymmetric carbon centers and geometric isomers (trans- or cis-), and optical isomers (enantiomers). All these isomers and mixtures thereof are also included within the scope of the present invention.

In the cancer immunotherapy composition according to the present invention, the compound represented by Formula 7 or a pharmaceutically acceptable salt thereof may be administered in various oral and parenteral dosage forms during clinical administration. When formulated, the preparation is prepared by using diluents or excipients such as commonly used fillers, extenders, binders, wetting agents, disintegrants, and surfactants. Solid preparations for oral administration include tablets, pills, powders, granules, capsules, and the like, and these solid preparations are prepared by mixing one or more compounds with at least one excipient, for example, starch, calcium carbonate, sucrose, or lactose gelatin, and the like. Also, in addition to simple excipients, lubricants such as magnesium stearate and talc are also used. Liquid preparations for oral administration include suspensions, oral solutions, emulsions, and syrups. In addition to water and liquid paraffin which are commonly used simple diluents, various excipients, for example, wetting agents, sweeteners, fragrances, and preservatives may be included. Preparations for parenteral administration include sterilized aqueous solutions, non-aqueous solvents, suspensions, and emulsions. As non-aqueous solvents and suspensions, propylene glycol, polyethylene glycol, vegetable oil such as olive oil, and injectable ester such as ethyl oleate may be used.

The cancer immunotherapy composition containing the compound represented by Formula 7 or pharmaceutically acceptable salts thereof as active ingredients can be administered parenterally, and parenteral administration can be performed by injection methods of subcutaneous injection, intravenous injection, intramuscular injection, or intrathoracic injection.

At this time, in order to formulate a dosage form for parenteral administration, the compound represented by Formula 7 or a pharmaceutically acceptable salt thereof is mixed with water along with a stabilizer or buffer to prepare a solution or suspension, and the solution or suspension can be administered in a dosage form of an ampoule or vial unit. The composition can be sterilized and/or can contain adjuvants such as preservatives, stabilizers, wetting agents, or emulsification accelerators, salts and/or buffers for adjusting osmotic pressure, and other therapeutically useful substances, and can be formulated according to mixing, granulating, or coating methods, which are methods in the related art.

Additional examples of dosage forms for oral administration include tablets, pills, hard/soft capsules, solutions, suspensions, emulsifiers, syrups, granules, elixirs, troches. These dosage forms contain diluents (for example, lactose, dextrose, sucrose, mannitol, sorbitol, cellulose, and/or glycine), lubricants (for example, silica, talc, stearic acid and magnesium or calcium salts thereof, and/or polyethylene glycol) in addition to the active ingredients. The tablets can contain binders such as magnesium aluminum silicate, starch paste, gelatin, methylcellulose, sodium carboxymethylcellulose, and/or polyvinylpyrrolidine and can contain, in some cases, disintegrants such as starch, agar, alginic acid, or sodium salts thereof, boiling mixtures and/or absorbents, colorants, flavoring agents, and sweeteners.

Also, the present invention provides a cancer immunotherapy adjuvant containing the interaction inhibitor of STING and TRIM29, the compound represented by Formula 7, isomers thereof, solvates thereof, hydrates thereof, or pharmaceutically acceptable salts thereof as active ingredients.

Also, the present invention provides an anticancer combination agent including the cancer immunotherapy adjuvant and the cancer immunotherapy agent.

The cancer immunotherapy adjuvant preferably increases the efficacy of the cancer immunotherapy agent and activates one or more immune factors selected from the group consisting of cytotoxic T cells, helper T cells, NK cells, and cytokines.

Also, the cancer immunotherapy adjuvant is preferably administered simultaneously or sequentially with the cancer immunotherapy agent, but the present invention is not limited thereto.

The cancer immunotherapy agents may be one or more selected from the group consisting of c-di-GMP (cyclic diguanylate), cGAMP, 3'3'-cGAMP, c-di-GAMP, c-di-AMP, and 2'3'-cGAMP, which can activate STING signaling, or anti-PD1, anti-PDL1, anti-CTLA4, anti-LAG3, anti-VISTA, anti-BTLA, anti-TIM3, anti-HVEM, anti-CD27, anti-CD137, anti-OX40, anti-CD28, anti-PDL2, anti-GITR, anti-ICOS, anti-SIRPα, anti-ILT2, anti-ILT3, anti-ILT4, anti-ILT5, anti-EGFR, anti-CD19, and anti-TIGIT, which are immune checkpoint inhibitors in the related art, but the present invention is not limited thereto.

The cancer immunotherapy adjuvant or the combination agent preferably have anticancer activity against one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, triple negative breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic Schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, blood cancer, and thymic cancer, but the present invention is not limited thereto.

Also, specific description of the cancer immunotherapy adjuvant and the combination agent according to the present invention is the same as the description of the "pharmaceutical composition for cancer immunotherapy" according to the present invention.

In addition, the present invention provides a method for screening cancer immunotherapy agent, including:
1) a step of preparing a vector including genes encoding a STING protein bound to a luminescent protein and a TRIM29 protein bound to a luminescent protein, respectively;
2) a step of transforming the vector of step 1) into a cell;
3) a step of treating the transformed cell of step 2) with a test substance; and
4) a step of comparing the cell treated with the test substance of step 3) with an untreated control group and selecting a substance that reduces the luminescence signal from the treated cell.

Also, the present invention provides a kit for screening cancer immunotherapy agent including a STING protein bound to a luminescent protein and a TRIM29 protein bound to a luminescent protein.

The STING protein bound to a luminescent protein of step 1) is obtained by binding LgBiT to the C-terminus of STING, and the TRIM29 protein bound to a luminescent protein is obtained by binding SmBiT to the C-terminus or the N-terminus of TRIM29. LgBiT and SmBiT do not interact directly with each other, but are bound only through interactions between linked proteins to perform the function of a luminescent enzyme.

The vector in step 1) refers to means for expressing a target gene in a host cell. The vector includes elements for expression of the target gene, and may include a replication origin, a promoter, an operator gene (operator), a transcription termination sequence (terminator), and the like and may additionally include selection markers for confirming appropriate enzymatic sites (for example, restriction enzyme sites) for introduction into the genome of the host cell and/or successful introduction into host cell, and/or ribosome binding site (RBS), for translation into proteins, IRES (internal ribosome entry site, and the like. The vector may additionally include transcription control sequences (for example, enhancers) other than the promoter.

In addition, the vector may be plasmid DNA, a recombinant vector, or other carrier known in the related art, and specifically may be linear DNA plasmid DNA, a recombinant non-viral vector, a recombinant viral vector, or an inducible gene expression vector system, and the recombinant viral vector may be retrovirus, adenovirus, adeno associated virus, helper-dependent adenovirus, herpes simplex virus, lentivirus, or vaccinia virus vector, but the present invention is not limited thereto.

Additionally, the transformed cells may be stem cells, progenitor cells, or animal cells, but are not limited thereto.

In addition, the components of the kit may be a host cell into which a vector including genes encoding a STING protein bound to a luminescent protein and a TRIM29 protein bound to a luminescent protein, respectively, are introduced, a medium for culturing the host cell, a container for culturing the host cell, software for analyzing a fluorescence level, a hardware system equipped with the software, and the like.

Meanwhile, the specific description of the method and kit for screening cancer immunotherapy agent of the present invention is the same as the description of the "pharmaceutical composition for cancer immunotherapy" according to the present invention.

Hereinafter, the present invention is described in detail with reference to examples and experimental examples.

However, the following examples and experimental examples specifically illustrate the present invention, and the content of the present invention is not limited by the examples and experimental examples.

### <Example 1> Preparation of kit and reagents

DMEM (Dulbecco's modified eagle medium) (11995-065), MEM (minimum essential medium) (11095-080), RPMI 1640 (11875-093), Opti-MEM (31985-070), FBS (fetal bovine serum) (16000044), antibiotic-antimycotic solution (15240062), and TrypLETM Express solution (12605-010) were purchased from Gibco, Invitrogen [Carlsbad, CA]. A PBS (Phosphate-buffered saline) buffer was purchased from WELGENE [Gyeongsan, Korea]. Compounds dissolved in DMSO (dimethylsulfoxide) for biological evaluation were prepared as the compounds. DMSO, cycloheximide, actinomycin D, MG132, sodium deoxycholate, IGEPAL CA-630, Tween-20, Na3VO4, NaF, Triton-X-100, ammonium persulfate, and *N,N,N',N'*-tetramethylethylenediamine were purchased from Sigma-Aldrich [St. Louis, MO].

Also, tris(hydroxymethyl)aminomethane was purchased from Acros Organics [Waltham, MA], NaCl was purchased from Samchun Chemical [Daejeon, Korea], and complete Tablets EDTA-free Protease inhibitor cocktail (04693132001) was purchased from Roche [Basel, Switzerland]. 2',3'-Cyclic GMP-AMP(cGAMP) was purchased from Invivogen [Pak Shek Kok, Hong Kong] or APExBIO [Houston, TX], NanoBiTTM PPI MCS starter kit (N2014) and a Nano-Glo live cell assay reagent (N2012) were purchased from Promega [Madison, WI], and an Ez-Cytox WST assay reagent was purchased from Daeil Bio Co. Ltd [Seoul, Korea] .

To measure the protein concentration in cell lysates, Micro BCATM protein assay kit was purchased from PIERCE [Waltham, MA], 3,3',5,5"-tetramethylbenzidine (TMB) and a substrate of horseradish peroxidase (HRP) for ELISA were purchased from Invitrogen [Carlsbad, CA], 30% Acrylamide-Bis solution (37.5:1, AC4004-050-00) and a lammeli sampling buffer (5× SDS-PAGE loading buffer, SF2002-110-00) were purchased from Biosesang [Seongnam, Korea]. Protein gel casters including a polyvinylidene difluoride (PVDF) membrane and western blot equipment were purchased from Bio-Rad Laboratories, Inc. [Hercules, CA], an Amersham ECL prime Western blotting detection system (RPN2232) and percoll were purchased from GE Healthcare Life Science [Chicago, IL], and RNeasy mini kit for RNA extraction was purchased from QIAGEN [Hilden, Germany].

Lipofectamine LTX and Plus reagents and lipofectamine RNAiMAX were purchased from ThermoFisher Scientific [Waltham, MA], all qPCR primers, siRNAs and Accupower Cyclescript RT premix (dT20) for reverse transcription were purchased from Bioneer, Inc [Daejeon, Korea], and a Kapa SYBR Green 2× ABI Prism reagent for qPCR was purchased from KAPA biosystems. Also, RBC lysis buffer was purchased from Biolegend [San Diego, CA], a Foxp3/Transcription factor staining buffer was purchased from eBioscience [San Diego, CA], 100-mm/150-mm cell culture dish, a transparent 96-well plate, a transparent 12-well plate, a transparent 6-well plate, a 96-well white flat bottom TC-treated microtest assay microplate (353296), and a breathable sealing film (Axygen BF-400-S) were purchased from CORNING [Glendale, AZ].

Also, 6-pin multi-blot replicator (VP408) for NanoBiT screening was purchased from V&P Scientific, Inc. [San Diego, CA], a Nunc Maxisorp 96-well ELISA plate (439454) was purchased from Costar, pCMV6-TRIM29-Myc-FLAG plasmid for Western blotting after TRIM29 overexpression was purchased from Origene [Rockville, MD], FLAG- or Myc-tagged plasmids for ELISA were manufactured from pcDNA3.1 vectors (pcDNA3.1-FLAG-STING and pcDNA3.1-TRIM29-Myc), and pRK5-HA-ubiquitin (WT and K48) plasmids were purchased from Addgene.

### <Example 2> Preparation of antibodies

Anti-β-actin (#4970), anti-STING (#13647, for western blotting), anti-phospho-STING (S365) (#72971), anti-TBK1/NAK1 (#3504), anti-phsopho-TBK1/NAK1 (S172) (#5483), anti-IRF3 (#4302), anti-phospho-IRF3 (S396) (#29047), HRP-conjugated anti-rabbit IgG [#7074], and HRP-conjugated anti-mouse IgG [#7076] antibodies were purchased from Cell Signaling Technology [Danvers, MA], an anti-FLAG M2 (F1804) antibody was purchased from Sigma-Aldrich [ST. Louis, MO], and anti-TRIM29/ATDC (B-2) (sc-166707, for detecting human TRIM29), anti-TRIM29/ATDC (C-2) (sc-376125, for detecting mouse TRIM29), and protein A-agarose beads (sc-2001) were purchased from Santa Cruz Biotechnology [Dallas, TX]. Also, anti-ubiquitin (ab134953) antibodies were purchased from Abeam [Cambridge, UK], and anti-HA-tag (51642-2-AP, for ELISA) and anti-STING (19851-1-AP, for FACS) antibodies were purchased from Proteintech [Rosemont, IL].

In addition, CD274(B7-H1, PD-L1)-APC, CD3(17A.2)-PE, CD4(RM45)-FITC, CD8α(53-6.7)-APC-Cy7, CD19(6D5)-BV650, CD11b(M1/70)-BV786, Ly6C(HK1.4)-PE-Cy7, Ly6G(1A8)-APC, CD45.2(104)-PerCP-Cy5.5, IFNγ(XMG1.2)-APC, CD279(29F.1A12)-APC, granzyme B(QA16A02)-FITC, and perforin(S16009A)-PE which were antibodies for flow cytometry were purchased from Biolegend [San Diego, CA], and Gr1(RB6-8C5)-BV510, CD49b(HMa2)-BV605, F4/80(T45-2342)-BV711, Foxp3(R16-715)-PE, TNF-α(MP6-XT22)-PE, and I-A/I-E(M5/11)-BV605 were purchased from BD Bioscience [San Jose, CA].

### <Example 3> Preparation of cell lines animal model

A431 human skin squamous cell carcinoma cells and CT26 murine colon cancer cells were obtained from the Korean Cell Line Bank. Raw264.7 murine macrophages and HEK293T human embryonic kidney cells were obtained from ATCC (American Type Culture Collections). Mouse bone marrow-derived cells were obtained from 8-week-old female, C57BL/6 mice, and OVA-specific CD8⁺ T cells (OT-I T cells) were obtained from 8-week-old OVA-specific CD8⁺ TCR transgenic mice (OT-I mice). Additionally, BALB/c mice were obtained from Jackson Laboratory [Bar Harbor, ME]. In addition, in the experiment, female mice which were 8 to 12 weeks old were used, and all mice, including wild-type BALB/c, were bred and managed in the animal facility of Seoul National University College of Medicine, and all animal experiments were performed with the approval of the Animal Care and Use Committee of Seoul National University Institute of Laboratory Animal Resources (Approval No. SNU-190621-3-13).

### <Example 4> Cell culture

Raw264.7 and HEK293T cells were cultured in DMEM including 10% (v/v) FBS and 1% (v/v) antibiotic-antimycotic solution, and A431 cells were cultured in MEM including 10% (v/v) FBS and 1% (v/v) antibiotic-antimycotic solution. Additionally, CT26 cells were cultured in an RPMI 1640 medium including 10% (v/v) FBS and 1% (v/v) antibiotic-antimycotic solution. Meanwhile, cells were maintained in a 100-mm cell culture dish in a 5% CO₂ incubator set at 37°C and humidified conditions.

### <Example 5> Experimental equipment and program

Absorbance measurements in 96-well plates for ELISA analysis and BCA analysis were performed with a BioTek Synergy HT microplate reader [Winooski, VT]. Luminescence measurement of white-bottom 96-well plates for NanoBiT screening was performed with a BioTeK Synergy HTX microplate reader [Winooski, VT]. A ChemiDocTM MP imaging system of Bio-Rad Laboratories, Inc. was used for chemical luminescence signal analysis for Western blotting analysis.

In addition, quantification of chemical luminescence signals was performed by using the ImageLab 6.0 program provided by BioRad [Hercules, CA]. RNA quantification was performed with NanoView from GE Healthcare [Chicago, IL]. Quantitative real-time PCR was performed with the StepOne Plus Real-Time PCR system from Applied Biosystems [Foster city, CA]. Graphs were analyzed with GraphPad Prism 8 [La Jolla, CA].

In addition, for flow cytometry experiments, cells were analyzed by using FACSCalibur or FACSAria II (NCIRF) from BD Biosciences [San Jose, CA] and FlowJo software from Tree Star [Ashland, OR].

### <Experimental Example 1> Screening for compound that inhibit interaction of STING and TRIM29

### <1-1> Construction of highly efficient physiological activity search method

To observe protein-protein interactions, a highly efficient physiological activity search method based on bimolecular luminescence complementation assay was constructed. As shown in FIG. 1, the two proteins LgBiT and SmBiT constituting the luminescent protein luciferase were introduced into the terminus parts of STING and TRIM29, respectively. LgBiT and SmBiT do not interact directly with each other, but are bound only through interactions between linked proteins to perform the function of a luminescent enzyme.

In order to obtain an interaction luminescence signal of STING and TRIM29 by introducing LgBiT and SmBiT into STING and TRIM29, a total of eight combinations of introducing LgBiT and SmBiT into the N-terminus and C-terminus of STING and TRIM29, respectively, as shown in FIG. 2 were attempted. Therefore, higher luminescence signals were confirmed in combinations of introducing LgBiT to the C-terminus of STING and SmBiT to the N-terminus of TRIM29, and LgBiT to the C-terminus of STING and SmBiT to the C-terminus of TRIM29, compared with a case where a protein that does not interact with STING, named HaloTag, was introduced together.

### <1-2> Discovery of interaction inhibitors of STING and TRIM29

Screening was performed in the following manner by using the system established in Experimental Example <1-1>.

Specifically, the HEK293T cell line was seeded in a 100-mm culture dish and cultured for one day. Thereafter, a combination of plasmids containing STING-LgBiT and SmBiT-TRIM29, respectively, or a combination of plasmids containing STING-LgBiT and HaloTag-SmBiT as a negative control was introduced into the cells and cultured for one additional day. Introduction of the plasmid was performed by using Lipofectamine LTX from ThermoFisher according to the protocol of the manufacturer. After culturing, the cells into which the plasmid was introduced were transferred to a white bottom 96-well plate, seeded, and cultured for one additional day. After treating each well with Nano-Glo live cell reagent of Promega Corporation according to the protocol of the manufacturer, luminescence signals were obtained while shaking every 5 minutes. Each well was treated with the compound when the signal was stabilized, and then a luminescence signal was obtained. Whether STING-TRIM29 interaction was inhibited was observed with a value obtained by standardizing this obtained luminescence signal to the luminescence signal of each well at the time of stabilization.

### <1-3> Confirmation of STING-TRIM29 interaction inhibition

Through the screening of Experimental Example <1-2>, oxopiperazine derivatives were confirmed that reduces the luminescence signal in a concentration-dependent manner by inhibiting the STING-TRIM29 interaction, as shown in FIG. 3 (FIG. 3).

In addition, the oxopiperazine derivatives are represented by [Formula 1] to [Formula 6] below.

### [Formula 1]

### 4-((R)-4-(4-hydroxybenzyl)-2-isopropyl-5-(4-methoxybenzyl)-3-oxo-3,4-dihydropyrazin-1(2H)-yl)-3-nitro-N-(((R)-tetrahydrofuran-2-yl)methyl)benzamide

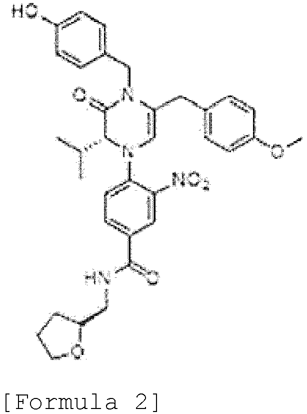

### 4-((R)-5-cyclopentyl-4-(4-hydroxybenzyl)-2-isopropyl-3-oxo-3,4-dihydropyrazin-1(2H)-yl)-3-nitro-N-(((R)-tetrahydrofuran-2-yl)methyl)benzamide

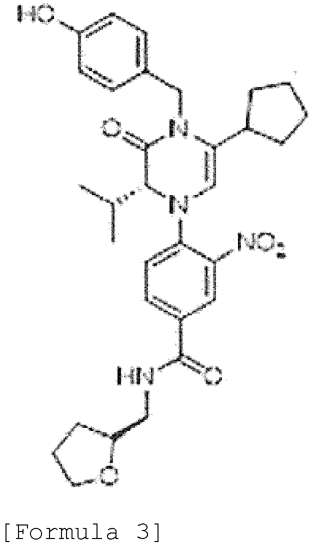

### (R)-4-(5-cyclopentyl-4-(4-hydroxybenzyl)-2-isopropyl-3-oxo-3,4-dihydropyrazin-1(2H)-yl)-N,N-diethyl-3-nitrobenzamide

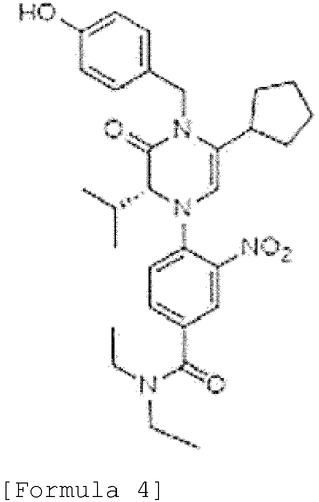

### (R)-4-(5-cyclopentyl-4-(4-hydroxybenzyl)-2-isopropyl-3-oxo-3,4-dihydropyrazin-1(2H)-yl)-N-isobutyl-3-nitrobenzamide

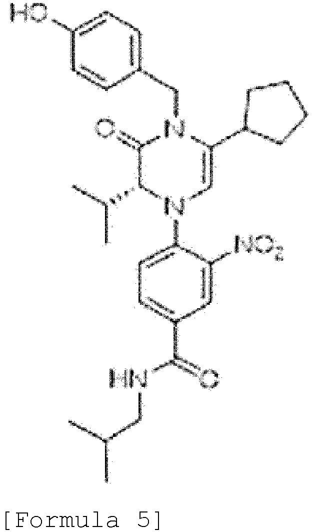

### 4-((S)-5-cyclopentyl-2-(4-fluorobenzyl)-4-(4-hydroxybenzyl)-3-oxo-3,4-dihydropyrazin-1(2H)-yl)-3-nitro-N-(((R)-tetrahydrofuran-2-yl)methyl)benzamide

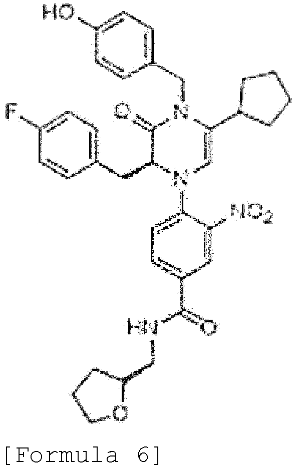

### 4-((R)-5-benzyl-4-(4-hydroxybenzyl)-2-isopropyl-3-oxo-3,4-dihydropyrazin-1(2H)-yl)-3-nitro-N-(((R)-tetrahydrofuran-2-yl)methyl)benzamide

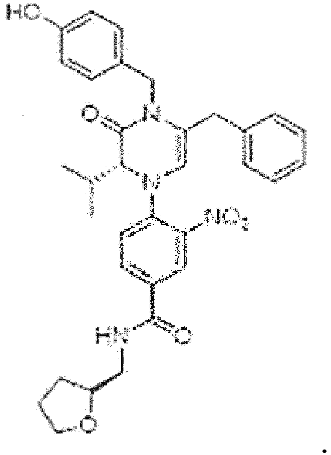

### <Experimental Example 2> Confirmation of inducing an increase in the intracellular amount of a STING protein by oxopiperazine derivatives

In order to confirm whether the oxopiperazine derivatives of Formulas 1 to 6 discovered in <Experimental Example 1> increase the intracellular amount of STING by inhibiting the STING-TRIM29 interaction, the intracellular amount of STING after compound treatment was confirmed through repeated Western blotting experiments.

First, the A431 cell line was seeded in a transparent 12-well plate and cultured for one day. Thereafter, the cells were treated with the compounds of Formulas 1 to 6, respectively, and then cultured for additional 24 hours. Finally, after supernatant culture medium of the cells was removed, the remaining supernatant was diluted and removed with PBS (phosphate buffer saline) to prepare a Western blotting sample. All procedures for preparing Western blotting sample were performed at 4°C. The cells were treated with RIPA lysis buffer (50 mM Tris-HCl pH 7.5, 150 mM NaCl, 1% (w/v) sodium deoxycholate, 2 mM Na3VO4, 5 mM NaF, protease inhibitor cocktail, and 1% (v/v) IGEPAL CA-630) for 15 minutes, the solution was collected, and centrifugation was performed at 13000 g for 15 minutes to obtain the supernatant. The protein concentration of the supernatant was measured by using the Micro BCATM protein assay kit (ThermoFisher) according to the protocol of the manufacturer. The concentration of each sample was adjusted to be the same by using RIPA lysis buffer, and then 5X SDS-PAGE loading buffer (Biosesang) was added according to the ratio of the manufacturer and treated at 95°C for five minutes. The treated samples were analyzed by SDS-PAGE, then transferred to a PVDF membrane, and cultured in a TBST solution containing 4% BSA for 30 minutes. The primary antibody was cultured at 4°C for one day or at room temperature for one hour, and then washed four times with the TBST solution at room temperature. Secondary antibodies were cultured at room temperature for one hour. The antibody solution was diluted in TBST solution containing 1% BSA according to the protocol of the manufacturer for each antibody. Finally, after four TBST washing processes, the solution was developed with an ECL (enhanced chemiluminescence) prime solution of Amersham, the chemical luminescence signal was measured by using ChemiDoc MP of Bio-Rad Laboratories, Inc., the obtained results were quantified by using the ImageLab 6.0 program, and the results were plotted by using the Prism 8.0 program.

As a result, a value by quantifying the band intensity obtained as a result of the above analysis is shown in FIG. 4, and it was confirmed that all the oxopiperazine derivatives of Formulas 1 to 6 increased the intracellular amount of STING.

### <Experimental Example 3> Confirmation of increase in immune activation efficacy of STING agonist of oxopiperazine derivatives

### <3-1> Confirmation of increase in immune activation efficacy of cGAMP by using qRT-PCR

In order to confirm whether the oxopiperazine derivatives of Formulas 1 to 6 increase the immune activation efficacy of the STING agonist cGAMP by increasing the amount of STING, the gene expression levels of the STING subcytokine interferon-beta (IFN-β), interleukin-6 (IL-6), interleukin-15 (IL-15), and interleukin-15 receptor alpha (IL-15Rα) were confirmed through quantitative real-time PCR (qRT-PCR) experiments.

Specifically, Raw264.7 cells were seeded in a transparent 12-well plate and cultured for one day. Thereafter, the cells were treated with cGAMP and the oxopiperazine derivatives of Formulas 1 to 6, respectively, and then cultured for six hours. Finally, after the supernatant culture medium of the cells was removed, the remaining supernatant was diluted with PBS (phosphate buffer saline) and removed to prepare qRT-PCR samples. mRNA was extracted from cells by using the RNeasy mini kit of QIAGEN according to the protocol of the manufacturer. After the concentration of RNA was measured by using Nanoview equipment of GE Healthcare, 1 µg RNA was quantified, and complementary DNA (cDNA) was synthesized by using the Accupower cyclescript RT premix dT20 (Bioneer) product according to the protocol of the manufacturer. The obtained cDNA was mixed with primer DNA capable of detecting GAPDH (control group), IFN-β, and IL-6 genes in Kapa SYBR Green 2X ABI Prism solution of Kapa Biosystems, Inc. according to the protocol of the manufacturer. The mixed samples were analyzed by using the StepOne Plus Real-Time PCR system (Applied Biosciences), and the results were plotted by using the Prism 8.0 program.

Meanwhile, the DNA base sequences of the primers used in the analysis are shown in Table 1 below.

**[Table 1]**

| qPCR primer | Sequence (5'-3') |
|---|---|
| mGAPDH_F (Sequence Number 1) | TGGGCTACACTGAGCACCAG |
| mGAPDH_R (Sequence Number 2) | GGGTGTCGCTGTTGAAGTCA |
| mIFNβ_F (Sequence Number 3) | AAGAGTTACACTGCCTTTGCCATC |
| mIFNβ_R (Sequence Number 4) | CACTGTCTGCTGGTGGAGTTCATC |
| mIL6_F (Sequence Number 5) | TCCAGTTGCCTTCTTGGGAC |
| mIL6_R (Sequence Number 6) | GTACTCCAGAAGACCAGAGG |
| mIL15_F (Sequence Number 7) | GAGACTTTCATCCCAGTTCG |
| mIL15_R (Sequence Number 8) | TTCCTTGCAGCCAGATTCTG |
| mIL15Rα_F (Sequence Number 9) | CCCACAGTTCCAAAATGACGA |
| mIL15Rα_R (Sequence Number 10) | GCTGCCTTGATTTGATGTACCAG |

As a result, as shown in FIG. 5, it was confirmed that all the oxopiperazine derivatives of Formulas 1 to 6 increased the expression of STING subcytokines IFN-β and IL-6 under the co-treatment with cGAMP.

In addition, as shown in FIG. 6, it was confirmed that the compound of Formula 1 also increased the expression of IL-15 and IL-15Rα, which are other cytokines known to be expressed downstream of STING, in addition to IFN-β and IL-6, in a time-dependent and concentration-dependent manner.

### <3-2> Confirmation of increase in immune activation efficacy of cGAMP using Western blotting

It was verified that the oxopiperazine derivative of Formula 1 increased the effect of STING sub-immune signaling pathway activation of cGAMP, through Western blotting in the same manner as in <Example 2> above.

As a result, as shown in FIG. 7, it was confirmed that, as STING was activated by cGAMP, the phosphorylation of downstream signaling proteins TBK1 and IRF3 increased, thereby increasing the expression of immune cytokines, and it was confirmed that the degree of phosphorylation further increased when administered in combination with the oxopiperazine derivative of Formula 1.

### <Experimental Example 4> Identification of degradation inhibition by TRIM29-mediated Lys48 polymerized multi-ubiquitination of STING

It was confirmed whether the oxopiperazine derivatives of Formulas 1 to 6 increased the intracellular amount by preventing the degradation of STING through TRIM29-mediated Lys48 polymerized multi-ubiquitination (K48 linkage-specific polyubiquitination). For this purpose, the compound of Formula 1 was selected as a representative compound, and as shown in FIG. 8, quantitative STING Lys48 polymerized multi-ubiquitination detection was performed based on the ELISA (Enzyme-linked immunosorbent assay) method by using transformed ubiquitin protein in which all lysine residues other than lysine residue 48 of a ubiquitin protein were replaced with arginine.

Specifically, HEK293T cells were seeded in a transparent 6-well plate and cultured for one day. Thereafter, pcDNA3.1-FLAG-STING (plasmid expressing STING linked with FLAG label), pcDNA3.1-TRIM29-Myc (plasmid expressing TRIM29 linked with Myc label), and pRK5-HA-Ubiquitin-K48 (plasmid expressing protein in which HA label is linked to a ubiquitin in which all lysine residues other than lysine residue 48 were replaced with arginine) were introduced and cultured for one day. At this time, the total amount of DNA introduced for each experimental condition was standardized by using the pUC19 empty vector. Introduction of the plasmid was performed by using Lipofectamine LTX from ThermoFisher according to the protocol of the manufacturer. After culturing for one day, the supernatant culture medium was removed, and the culture medium containing MG132 and the compound of Formula 1 was treated and cultured for additional three hours. At this time, only the effect of the compound was quantitatively detected in a manner of using an analysis value of the sample that was not treated with MG132, a proteasome inhibitor that prevents the removal of ubiquitinated proteins, as a control group and subtracting the analysis value from an analysis value of the sample that was treated with MG132. Finally, after removing the supernatant culture medium of the cells, ELISA treatment samples were prepared by diluting and removing the remaining supernatant with PBS (phosphate buffer saline). The cells were treated with the ELISA lysis buffer (25 mM Tris-HCl pH 7.5, 150 mM NaCl, 10 mM EDTA, 10 mM N-ethylmaleimide, 1 mM Na3VO4, 1 mM NaF, protease inhibitor cocktail, and 1% (v/v) Triton-X-100) for 15 minutes, the solution was collected and centrifuged at 13000 g for 15 minutes to obtain the supernatant. The protein concentration of the supernatant was measured by using Bradford assay reagent. The concentrations of the samples were equally set to 400 µg/mL by using the ELISA lysis buffer. To confirm the quantitative concentration, a 5X SDS-PAGE loading buffer (Biosesang) was added at the ratio of the manufacturer to a portion of the sample, and then the resultant was treated at 95°C for five minutes and analyzed by using the western blotting method described above.

For ELISA analysis, a FLAG antibody (Sigma) was diluted 1:3000 (volume ratio) in PBS and treated at 100 µL per well on a MaxiSorp ELISA plate of Nunc A/S for one day at 4°C. Thereafter, the supernatant was removed, and then the cells were washed three times with 200 µL per well with the ELISA washing solution (PBS solution containing 0.05% Tween-20). Then, 300 µL per well of the PBS solution containing 5% BSA was treated at room temperature for two hours. The resultant was washed five times with ELISA washing solution and then treated with 100 µL of the previously prepared sample per well at room temperature for three hours. The resultant was washed 10 times with the ELISA washing solution and then treated at room temperature for one hour with 100 µL per well of an HA antibody (Proteintech) diluted 1:2000 (volume ratio) in PBS containing 1% BSA. The resultant was washed five times with the ELISA washing solution and then treated at room temperature for one hour with 100 µL per well of an HRP-polymerized rabbit IgG antibody (CST) diluted 1:2000 (volume ratio) in PBS containing 1% BSA. The resultant was washed five times with the ELISA washing solution and then treated at room temperature for 10 minutes with 100 µL of a 3,3',5,5"-tetramethylbenzidine (TMB) solution per well, a substrate of an HRP enzyme, to perform color development. The color development reaction was completed by dispensing 50 µL of 2N H₂SO₄ per well, the absorbance was measured at 450 nm by using a Synergy HT microplate reader device of BioTek Instruments, and the results were plotted by using the Prism 8.0 program.

As a result, as shown in FIG. 8, the degree of Lys48 polymerized multi-ubiquitination of STING was able to be detected by capturing FLAG-labeled STING and then detecting the HA label, which selectively labels only lysine 48 polymerized multi-ubiquitination, with an antibody. As a result of treatment with the representative compound of Formula 1, it was confirmed that TRIM29-mediated lysine 48 polymerized multi-ubiquitination of STING was inhibited. In addition, it was confirmed through Western blotting that the concentration of each control group and the sample of the compound of Formula 1 was well quantified.

In addition, in order to cross-validate the results of the ELISA experiment, it was confirmed whether the STING increasing effect of the compound of Formula 1 was disappeared when ubiquitinated proteins were prevented from being degraded by treatment with MG132.

As a result, as shown in FIG. 9, using the Western blotting method, it was confirmed that STING was not increased when MG132 was treated with the compound of Formula 1.

In addition, in order to exclude the possibility that the increase in STING expression by the compound of Formula 1 can occur through cellular mechanisms other than ubiquitin-mediated reactions, the transcription inhibitor actinomycin D (ActD) and the ribosome translation inhibitor cycloheximide (CHX) were co-treated with the compound of Formula 1, and whether the STING increase effect was still maintained was confirmed by using the Western blotting method.

As a result, as shown in FIG. 10, it was confirmed that the compound of Formula 1 still increased the intracellular amount of STING even under conditions in which the cellular transcription and translation mechanisms were inhibited.

### <Experimental Example 5> Confirmation of binding of oxopiperazine derivatives to STING

It was verified whether the oxopiperazine derivative of the present invention was bound to STING among STING and TRIM29 to inhibit interaction thereof, based on the compound of Formula 1, by the cellular thermal shift assay (CETSA) and the isothermal dose response fingerprint (ITDRF) which were major technologies for verifying the interaction between small molecule compounds and target proteins. (D. M. Molinaetal., Science2013, 341, 84-87.)

To verify the target of the compound of Formula 1 using the CETSA technique, the human A431 cell lines and mouse Raw264.7 cell lines were seeded on a 100-mm or 150-mm culture dish and cultured for one day. Thereafter, cells were collected in a 50-mL tube and treated with culture media including 1.5625 µM, 3.125 µM, 6.25 µM, 12.5 µM, 25 µM, 50 µM, and 100 µM of the compound of Formula 1 for two hours. Then, the cells were dispensed in equal amounts, treated at the temperature shown in FIG. 11 (A431) or FIG. 13 (Raw264.7) for three minutes, respectively, and stabilized at 25°C for three minutes. The cells treated at the above temperature were centrifuged at 1000 g for three minutes, the supernatant was removed, and the cells were washed with PBS and further centrifuged to remove all the supernatant. The washed cells were treated with a PBS-N solution (PBS containing 0.4% IGEPAL CA-630) and equalized, and then the cells were lysed by repeating the rapid freezing-thawing process a total of three times by using liquid nitrogen. Afterwards, centrifugation was performed at 20000 g for 20 minutes at 4°C, the supernatant was transferred to a new container, and each concentration was measured by using the Micro BCA TM protein assay kit (ThermoFisher) according to the protocol of the manufacturer. The concentration of each sample was equally adjusted by using the RIPA lysis buffer, the 5X SDS-PAGE loading buffer (Biosesang) was added according to the ratio of the manufacturer, and the resultant was treated at 95°C for 5 minutes and analyzed by using the Western blotting method described above.

As a result, as shown in FIG. 11, it was confirmed through CETSA results in A431, the human cell line, that the compound of Formula 1 induced thermal destabilization by selective binding only to STING. Also, as shown in FIG. 12, it was cross-verified through the ITDRF results that the compound of Formula 1 reduced the thermal stability of STING at high concentrations in a concentration-dependent manner without affecting TRIM29.

In addition, as shown in FIG. 13, it was confirmed through CETSA results in Raw264.7, the mouse cell line, that the compound of Formula 1 induced thermal destabilization by selective binding only to STING. Also, as shown in FIG. 14, it was cross-verified through the ITDRF results that the compound of Formula 1 reduced the thermal stability of STING at high concentrations in a concentration-dependent manner without affecting TRIM29.

### <Experimental Example 6> Confirmation of effect of oxopiperazine derivatives on increasing intracellular amount of STING

It was verified whether the oxopiperazine derivatives increased the efficacy of STING agonists and other cancer immunotherapy, including immune checkpoint inhibitors. First, it was verified through flow cytometry and Western blotting whether the oxopiperazine derivatives increase the amount of STING.

To measure the intracellular amount of STING through flow cytometry, Raw264.7 cells were seeded in a transparent 6-well plate and cultured for one day. Thereafter, the cells were treated with a solution, in which the compound of Formula 1 is diluted in a culture medium without FBS, and cultured for 24 hours. Finally, the supernatant culture medium of the cells was removed, then the remaining supernatant was diluted and removed with PBS (phosphate buffer saline), and the cells were separated by treatment with a PBS solution containing 2 mM EDTA for five minutes and further treated with the culture medium and collected. After centrifugation of the collected cells, the supernatant was removed, the cells were redistributed in FACS solution (Hank's balanced salt solution (HBSS) including 2% horse serum and 0.05% NaN₃), and the number of cells was measured. 1×10⁶ cells for each sample were collected and centrifuged, the supernatant was removed, and the cells were washed once with PBS. The cells were fixed by treating with a 4% paraformaldehyde (PFA) solution at room temperature for 10 minutes, treated with Triton solution (PBS solution including 0.1% BSA and 0.5% Triton-X-100), and centrifuged, and then the supernatant was removed. The STING primary antibody (Proteintech) was treated at room temperature for two hours. Then, the Triton solution was added, the centrifugation was performed, the supernatant was removed, and the resultant was treated with FITC-polymerized secondary antibodies (Proteintech, FITC-conjugated affinipure goat anti-rabbit IgG) or an isotype control group (Zymed) for one hour at room temperature. Finally, the Triton solution was added, centrifugation was performed, the supernatant was removed, the cells were redistributed into 200 µL of the Triton solution and analyzed by using a FACSCalibur instrument from BD Bioscience, and the results were quantitatively analyzed by using the FlowJo program from Tree Star, Inc.

As a result, as shown in FIG. 15, when the cells were treated with the compound of Formula 1, the fluorescence labeling intensity of intracellular STING was increased in a concentration-dependent manner without any change in the isotype control group. In addition, as shown in FIG. 16, it was cross-verified by using Western blotting that the intracellular amount of STING in Raw264.7 cells increased by the compound of Formula 1 in a concentration-dependent manner. Also, as shown in FIG. 17, it was also verified through the qRT-PCR method that the increase of STING in Raw264.7 cells was not related to RNA transcription.

### <Experimental Example 7> Confirmation of effect of increasing antigen presentation efficacy of BMDC by STING agonist of oxopiperazine derivatives

It was confirmed that the compound of Formula 1 increased the antigen presentation efficacy of bone-marrow derived dendritic cells (BMDC) of a STING agonist to cytotoxic T cells (CD8⁺ T cells) .

Specifically, mouse bone marrow-derived cells were obtained from the femur and tibia of 8-week-old female C57BL/6 mice. 2×10⁶ of cells were cultured in 2 mL of a MLR medium (RPMI 1640 medium supplemented with 10% FBS, 1% 1M HEPES, 1% glutamine, and 1% antibiotic-antimycotic) supplemented with 10 ng/mL mGM-CSF and 5 ng/mL IL-4 to be differentiated into dendritic cells (BMDC). On the third day from the start of cell culturing, 2 mL of the MLR medium was added. Additionally, on the sixth day, 2×10⁶ dendritic cells were treated with 50 µg/mL OVA protein and/or 100 ng/mL lipopolysaccharide (LPS), and cGAMP and/or the compound represented by Formula 1 at various concentrations. Thereafter, the cells were cultured at 37°C in a 5% CO₂ incubator for 24 hours, and then flow cytometry of dendritic cell activation markers (CD80, CD86, MHC II) was performed. Subsequently, 5×10⁴ dendritic cells were incubated with 2×10⁵ CFSE-labeled OT-1 T cells (OVA-specific CD8⁺ T cells) to observe OT-1 T cell proliferation.

As a result, as shown in FIG. 18, it was confirmed that when OVA antigen was directly treated, the compound of Formula 1 promoted cell proliferation in a concentration-dependent manner by increasing the activation of BMDC by cGAMP and the antigen presentation efficacy from BMDC to OT-1 T cells.

### <Experimental Example 8> Confirmation of cross-antigen presentation ability of BMDC according to simultaneous treatment of oxopiperazine derivative and cGAMP

In the same manner as <Experimental Example 7> above, mouse bone marrow-derived cells were obtained and differentiated. On the sixth day, 2×10⁶ dendritic cells (BMDC) were co-cultured with 2×10⁶ of MC38 or MC38-OVA tumor cells frozen-thawed three times and treated with cGAMP and/or the compound of Formula 1 in various concentrations. The cells were cultured at 37°C in a 5% CO₂ incubator for 24 hours, and then flow cytometry of dendritic cell activation markers (CD80, CD86, MHC II) was performed. Then, 5×10⁴ cells were incubated with 2× 10⁵ CFSE-labeled OT-1 T cells (OVA-specific CD8⁺ T cells) to observe OT-1 T cell proliferation.

As a result, as shown in FIG. 19, also when the MC38 cell lines (MC38-OVA), that continuously expressed OVA antigen, and MC38 cell lines of the control group were killed and then treated, selectivity was confirmed by demonstrating that the compound of Formula 1 promoted cell proliferation by increasing, in a concentration-dependent manner, activation of BMDC by cGAMP and antigen presentation efficacy from BMDC to OT-1 T cells and that such an effect did not occur at all under conditions of MC38 control group.

### <Experimental Example 9> Confirmation of antigen-specific cytotoxicity of OT-I T Cells

In the same manner as <Experimental Example 7> above, mouse bone marrow-derived cells were obtained and differentiated. On the sixth day, 2×10⁶ dendritic cells were treated with cGAMP and/or the compound of Formula 1 in various concentrations in the presence of 50 ug/mL of an OVA protein. After incubation at 37°C in a 5% CO₂ incubator for 24 hours, 5×10⁴ cells were incubated with 2 ×10⁵ CFSE-labeled OT-1 T cells for one day to activate OT-I T cells. Effector cells, namely, activated OT-I T cells, were co-cultured with 2×10⁵ target cells, namely, MC38 and MC38-OVA tumor cells irradiated with 100 Gy (1×10⁵ MC38 tumor cells and 1×10⁵ MC38-OVA tumor cells expressing RFP) in various "effector:target" ratios (1:1 or 3:1). Then, after culturing at 37°C in a 5% CO₂ incubator for 24 hours, OVA-specific cytotoxicity of OT-I T cells was confirmed by using FACSCalibur and FlowJo software.

As a result, as shown in FIG. 20, it was confirmed that OT-1 T cells activated through the experiment of FIG. 19 selectively killed only cell lines expressing OVA.

### <Experimental Example 10> Analysis of T cell death when treating CD8⁺ T cells with cGAMP and/or oxopiperazine derivatives

CD8⁺ T cells were obtained from the lymph nodes of C57BL/6 mice and separated by using MACS. CD8⁺ T cells were activated on plates pre-coated with 1 µg/mL anti-CD3 and 0.5 µg/mL of anti-CD28 in the presence of 5 ng/mL of IL-2. After incubation for 24 hours in a 5% CO₂ incubator at 37°C, activated CD8⁺ T cells were treated with cGAMP and/or the compound of Formula 1 in various concentrations and incubated for additional 24 hours. The cells were harvested and stained with Annexin V/7-AAD to observe cell death. Data shown refer to at least three independent experiments.

As a result, as shown in FIG. 21, it was confirmed that the compound of Formula 1 did not induce or promote the death of cytotoxic T cells under all the conditions of treating the compound of Formula 1 alone or in combination with cGAMP compared to a case of treating with cGAMP.

### <Example 11> Confirmation of effect of increasing immune anticancer efficacy of cGAMP by compound of Formula 1 in in vivo syngeneic mouse model

### <11-1> Confirmation of tumor growth inhibitory effect

2×10⁵ CT26 cell lines were prepared in 50 µL culture medium and subcutaneously injected. Thereafter, as shown in FIG. 22, when the size of the injected tumor reached 100 mm3, cGAMP and the compound of Formula 1 were directly injected into the tumor. The cGAMP solution was manufactured with a final composition of PEG400:distilled water = 40:60, and 20 µL was injected each time. The solution of the compound of Formula 1 was manufactured with the composition of DMSO:PEG400:distilled water = 2:40:58, and 20 µL was injected each time. The cGAMP solution was injected once every two days, four times in total, and the solution of the compound of Formula 1 was injected once every four days, two times in total, starting with the second administration of cGAMP. The size of the tumor was measured by using calipers every two days, and the volume thereof was calculated as (length × width × height/2). The body weight of the mice was also measured every two days.

As a result, as shown in FIG. 23, it was confirmed that tumor growth was inhibited and the weight of the tumor extracted on the final day was reduced in the experimental group in which cGAMP (cG) and the compound of Formula 1 were treated in combination, compared with the control group, and it was also confirmed that the tumor growth was inhibited in a concentration-dependent manner when administered in combination with the compound of Formula 1 compared with the experimental group of the single treatment with cGAMP.

Additionally, as shown in FIG. 24, it was confirmed that a significant change in body weight or signal of toxicity was not observed regardless of the cases where cGAMP and the compound of Formula 1 were treated individually or in combination.

### <11-2> Confirmation of effect of increasing immune activation and immune anticancer efficacy

In order to verify immune cell infiltration into the tumor tissue and the degree of activation of the infiltrated immune cells, cGAMP (cG) and the compound of Formula 1 were treated individually or in combination, as shown in FIG. 25, the tumor was extracted after two days since the final treatment, and the flow cytometry was performed. The tumors were extracted after the mice were euthanized with carbon dioxide. The tumor tissues were divided into small pieces with a razor blade, treated with the DMEM solution including 2% horse serum, 0.5 mg/mL of collagenase D, and 40 ug/mL of DNase I for one hour at 37°C, and permeated through a 40-um filter, thereby being separated into individual cells. Tumor-infiltrating leukocytes (TILs) were separated from the separated cells by percoll concentration-gradient centrifugation, and red blood cells were removed by treatment with red blood cell lysis solution. The obtained cells were redistributed in the FACS solution described above, and FcγRII/111 receptors of individual immune cells were neutralized by treatment with the antimouse CD16/CD32 Abs (clone 2.4G2) antibody at 4°C for 10 minutes. The treatment with primary antibodies for individual cell differentiation were performed at 4°C for 30 minutes. The treated cells were redistributed into a PBS solution after centrifugation and then further centrifuged to remove the supernatant. The cells were fixed by the treatment with 4% paraformaldehyde (PFA) solution at room temperature for 10 minutes, treated with Triton solution (PBS solution including 0.1% BSA and 0.5% Triton-X-100), and centrifuged, and then the supernatant was removed. Cytokine (IFNγ(XMG1.2)-APC, Perforin(S16009A)-PE, granzyme B(QA16A02)-FITC, Biolegend) primary antibodies were treated at room temperature for two hours. Finally, the Triton solution was added, centrifugation was performed, the supernatant was removed, the cells were redistributed into 200 µL of the Triton solution and analyzed by using a FACSCalibur device from BD Bioscience, and the results were quantitatively analyzed by using the FlowJo program from Tree Star, Inc.

As a result, as shown in FIG. 26, it was verified that the environment of the entire immune response was activated by confirming that, in terms of the ratio of hematopoietic stem cell-derived cells, namely, CD45 positive (CD45⁺) cells representing total TIL, under the condition of treating cGAMP and the compound of Formula 1 in combination, the infiltration of the total T cells and cytotoxic T cells into the tumor was increased, and the total ratio of myeloid-derived suppressor cells (MDSC), which were cells that inhibited immune response, was decreased. In addition, it was confirmed, from the increase in the ratio of cytokine (TNF-α) and activating factors (granzyme B, Perforin), that the activity of infiltrated cytotoxic T cells (CD8⁺ T cells) was increased.

### <11-3> Confirmation of STING expression level in immune cell inside tumor

In order to investigate that STING increase in immune cells inside a tumor by the compound of Formula 1 is at the basis of increase in the immune activation and the immune anticancer efficacy, as shown in FIG. 27, the tumor was treated with the compound of Formula 1 alone, the tumor was extracted after one day since the final treatment, and the amount of STING inside thereof was confirmed by flow cytometry. As described above, after euthanasia with carbon dioxide, the tumor was extracted, TIL was separated, and intracellular STING staining was performed in the same manner as in <Example 6>.

As a result, as shown in FIG. 28, it was confirmed that the amount of STING was increased in antigen-presenting cells represented by tumor-associated macrophages (TAM) and dendritic cells (DC). In addition, it was confirmed that the effect of increasing STING was more noticeably exhibited in M1 macrophages, which directly activated the immune response, compared to M2 macrophages among all tumor-associated macrophage.

### <11-4> Confirmation of anti-cancer immune system construction effect

It was verified whether, under the treatment of cGAMP and the compound of Formula 1 in combination, in addition to the results of increasing the antigen presentation efficacy that were confirmed in FIGS. 18 to 21, the anticancer immune system was constructed throughout the organism also in the animal model, so that activated cytotoxic T cells were able to remove cancer cells in other locations to which drugs are not injected. For this purpose, as shown in FIG. 29, the primary tumor was transplanted first, then the secondary tumor was transplanted after four days, and drug administration was performed only to the primary tumor when the size of each tumor reached 100 mm³ and 20 mm³.

As a result, as shown in FIG. 30, similar to the results in FIG. 23, it was confirmed that the tumor growth was inhibited and the final tumor mass was reduced in the primary tumor under the treatment in combination with the compound of Formula 1, compared with the single administration of cGAMP (cG). Furthermore, it was finally confirmed that tumor death also effectively occurred in the secondary carcinomas to which the drug was not directly injected. Therefore, from the results, it was confirmed that the compound of Formula 1 effectively promotes the construction of the anticancer immune system in the entire organism.

### <11-5> Confirmation of effect of treatment of compound of Formula 1 and immune checkpoint therapeutic agent in combination

It was confirmed whether the efficacy can be increased in case of treating in combination with existing immune checkpoint therapeutic agents from the efficacy of constructing the systemic anticancer immune system of the compound of Formula 1. For this purpose, as shown in FIG. 31, a PD-1 antibody, an immune checkpoint therapeutic agent, was intravenously administered once every three days, the compound of Formula 1 was directly injected into the cancer once every four days, and then tumor growth and weight were measured.

As a result, as shown in FIG. 32, in case of treating the PD-1 antibody and the compound of Formula 1 in combination, it was confirmed that tumor growth and final tumor weight decreased in a concentration-dependent manner compared to single treatment with the PD-1 antibody.

## Claims

1. A cancer immunotherapy composition containing, as an active ingredient, an interaction inhibitor of stimulator of interferon genes (STING) and tripartite motif-containing protein 29 (TRIM29).

2. The cancer immunotherapy composition according to claim 1, wherein the interaction inhibitor is bound to STING.

3. The cancer immunotherapy composition according to claim 1, wherein the interaction inhibitor inhibits STING degradation through TRIM29-mediated Lys48 polymerized multi-ubiquitination of STING (K48 linkage-specific polyubiquitination).

4. The cancer immunotherapy composition according to claim 1, wherein the interaction inhibitor upregulates STING.

5. The cancer immunotherapy composition according to claim 1, wherein the interaction inhibitor increases activity of a STING agonist.

6. The cancer immunotherapy composition according to claim 1, wherein the interaction inhibitor activates one or more immune factors selected from the group consisting of cytotoxic T cells, helper T cells, NK cells, and cytokines.

7. The cancer immunotherapy composition according to claim 1, wherein the cancer immunotherapy agent prevents or treats one or more cancers selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, triple negative breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic Schwannoma, pancreatic cancer, salivary gland cancer, Kaposi's sarcoma, Paget's disease, tonsil cancer, squamous cell carcinoma, lung adenocarcinoma, lung cancer, squamous cell carcinoma of lung, skin cancer, anal cancer, rhabdomyosarcoma, laryngeal cancer, pleura cancer, blood cancer, and thymic cancer.

8. A cancer immunotherapy composition comprising, as active ingredients:
a compound represented by Formula 7, an isomer thereof, a solvate thereof, a hydrate thereof, or pharmaceutically acceptable salt thereof: 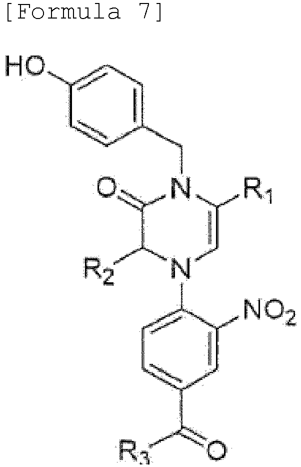
wherein, R₁ is cycloalkyl, aryl, or aryl-alkyl, where aryl may be optionally substituted with alkoxy;
R₂ is alkyl, or aryl-alkyl, where aryl may be optionally substituted with halogen; and
R₃ is amino, alkylamino, di-alkylamino, or heterocycloalkyl-alkylene-amino.

9. The cancer immunotherapy composition according to claim 8, wherein Formula 7 is any one of compounds represented by Formulas 1 to 6: 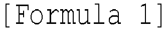 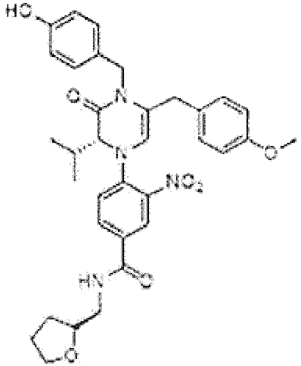 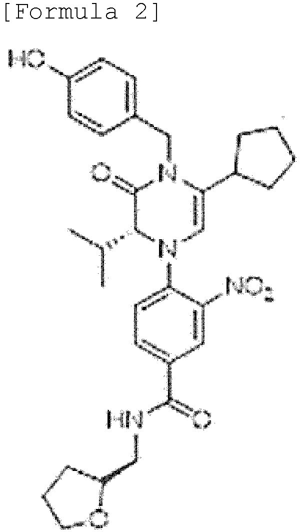 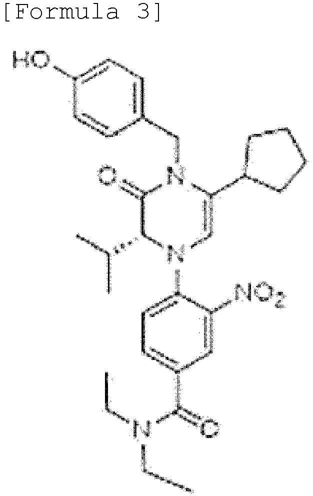 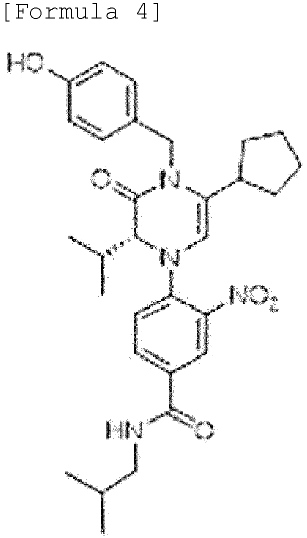 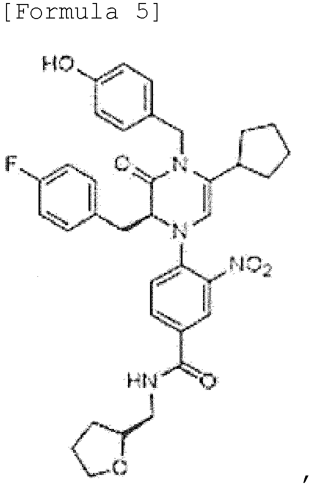 and

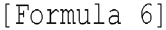

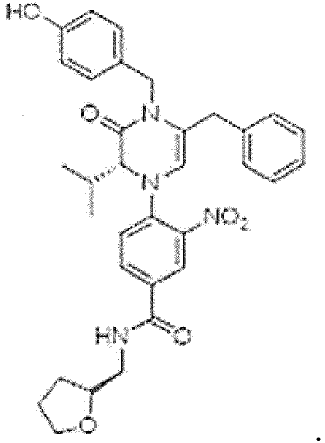

10. The cancer immunotherapy composition according to claim 8, wherein the compound inhibits interaction of STING and TRIM29.

11. The cancer immunotherapy composition according to claim 8, wherein the compound inhibits STING degradation through TRIM29-mediated Lys48 polymerized multi-ubiquitination of STING to upregulate STING or increase an amount of a STING protein.

12. The cancer immunotherapy composition according to claim 8, wherein the compound activates one or more immune factors selected from the group consisting of cytotoxic T cells, helper T cells, NK cells, and cytokines.

13. A cancer immunotherapy adjuvant comprising, the interaction inhibitor according to claim 1, the compound represented by Formula 7 according to claim 8, an isomer thereof, a solvate thereof, a hydrate thereof, or a pharmaceutically acceptable salt thereof as an active ingredient.

14. The cancer immunotherapy adjuvant according to claim 13, wherein the cancer immunotherapy adjuvant increases efficacy of a cancer immunotherapy agent.

15. The cancer immunotherapy adjuvant according to claim 13, wherein the cancer immunotherapy adjuvant is administered simultaneously or sequentially with a cancer immunotherapy agent.

16. The cancer immunotherapy adjuvant according to claim 15, wherein the cancer immunotherapy agent is one or more selected from the group consisting of c-di-GMP(cyclic diguanylate), cGAMP, 3'3'-cGAMP, c-di-GAMP, c-di-AMP, 2'3'-cGAMP, anti-PD1, anti-PDL1, anti-CTLA4, anti-LAG3, anti-VISTA, anti-BTLA, anti-TIM3, anti-HVEM, anti-CD27, anti-CD137, anti-OX40, anti-CD28, anti-PDL2, anti-GITR, anti-ICOS, anti-SIRPα, anti-ILT2, anti-ILT3, anti-ILT4, anti-ILT5, anti-EGFR, anti-CD19, and anti-TIGIT.

17. An anticancer combination agent comprising a cancer immunotherapy agent, and the cancer immunotherapy adjuvant according to claim 13.

18. A method for screening cancer immunotherapy agent, comprising:
1) a step of preparing a vector including genes encoding a STING protein bound to a luminescent protein and a TRIM29 protein bound to a luminescent protein, respectively;
2) a step of transforming the vector of step 1) into a cell;
3) a step of treating the transformed cell of step 2) with a test substance; and
4) a step of comparing the cell treated with the test substance of step 3) with an untreated control group and selecting a substance that reduces the luminescence signal from the treated cell.

19. The method for screening cancer immunotherapy agent according to claim 18, wherein the STING protein bound to a luminescent protein of step 1) is obtained by binding LgBiT to a C-terminus of STING.

20. The method for screening cancer immunotherapy agent according to claim 18, wherein the TRIM29 protein bound to a luminescent protein of step 1) is obtained by binding SmBiT to a C-terminus of TRIM29 or binding SmBiT to a N-terminus of TRIM29.

21. The method for screening cancer immunotherapy agent according to claim 18, wherein the cancer immunotherapy agent upregulates STING by regulating interaction of STING and TRIM29.

22. A kit for screening cancer immunotherapy agent, comprising:
a STING protein bound to a luminescent protein; and
a TRIM29 protein bound to a luminescent protein.
